# EUROPEAN PATENT SPECIFICATION

(11) **EP 1 640 016 B1**
(45) Date of publication and mention of the grant of the patent: **02.01.2008**
(21) Application number: 04022522.9
(22) Date of filing: 22.09.2004
(51) Int. Cl.: A61K 36/8965, A61K 125/00, A61K 135/00, A61P 7/10

(54) **Saponin-free extracts of Asparagus Officinalis L., their preparation and their use**
Verwendung und Herstellung von saponinfreien Spargelextrakten
Préparation et utilisation d'extraits d'asperges sans saponines

(43) Date of publication of application: 29.03.2006
(73) Proprietor: Plantina Entwicklungs GmbH, 82418 Murnau (DE)
(72) Inventor: Schnee, Uwe, Dr., 82418 Murnau (DE)
(74) Representative: Koepe, Gerd L.

(56) References cited:
- US-A1- 2003 181 395
- DATABASE WPI Section Ch, Week 198851 Derwent Publications Ltd., London, GB; Class D13, AN 1988-363978 XP002318108 & JP 63 273462 A (MARUTOMO KK) 10 November 1988 (1988-11-10)
- HAZEBROEK J P ET AL: "ALLELOPATHIC SUBSTANCES IN ASPARAGUS ROOTS EXTRACTION CHARACTERIZATION AND BIOLOGICAL ACTIVITY" JOURNAL OF THE AMERICAN SOCIETY FOR HORTICULTURAL SCIENCE, vol. 114, no. 1, 1989, pages 152-158, XP009044082 ISSN: 0003-1062
- HARTUNG A C ET AL: "ISOLATION AND CHARACTERIZATION OF PHYTOTOXIC COMPOUNDS FROM ASPARAGUS ASPARAGUS-OFFICINALIS L. ROOTS" JOURNAL OF CHEMICAL ECOLOGY, vol. 16, no. 5, 1990, pages 1707-1718, XP009044085 ISSN: 0098-0331
- SHAO Y ET AL: "Steroidal saponins from Asparagus officinalis and their cytotoxic activity." PLANTA MEDICA. JUN 1997, vol. 63, no. 3, June 1997 (1997-06), pages 258-262, XP009044083 ISSN: 0032-0943

## Description

The present invention relates to a saponine-free extract from roots and/or shoots of Asparagus Officinalis L.. The invention also relates to the preparation of saponine-free extracts from roots and/or shoots of Asparagus Officinalis L. and to their use for the manufacture of a medicament.

Asparagus Officinalis L. is traditionally cultivated in a temperate climate as food and medicinal plant for at least two thousand years. There are two types of asparagus produced in the world: White asparagus in which the shoots, which are young stems, are harvested from raised beds just when appearing on the soil; and green asparagus in which the shoots are harvested at the ground level.

The underground parts, i.e. the asparagus roots, are traditionally used as a diuretic in the dropsy disease. This diuretic property has been recommended to be made use of by ancient authors as Dioscoridos, Plinius, Celsius, and others. In the phytotherapy Asparagus roots are used in the form of cut rhizomes for teas as well as preparations having other galenical forms for internal use, for example in the form of a dried powder. The fields of application range from the irrigation therapy for inflammatory diseases of the urinary tract to the prevention of renal gravel and kidney stones, respectively. In addition, the powdered Asparagus roots are helpful in the weight reduction through augmented aquarese and as concomitant therapy in treating light essential hypertension. Up to now, any therapeutic effect of Asparagus shoots is unknown.

Beyond water and vegetable proteins, the Asparagus plant mainly contains amino acids, phenols, fats, steroids and saccharides as well as sodium and potassium as major trace elements. A typical characteristic of Asparagus is its saponine content. To date, many saponines have been isolated and characterised from Asparagus. They are all of the furastanol and the spirostanol types and belong to the steroidal saponines.

To date, it is still unclear, which of said components has a pharmacological effect. It is assumed, that the saponines, on the one hand, and/or the high potassium content, on the other hand, are responsible for the diuretic and, thus, the aquaretic effect.

Saponines are a group of compounds which are found in almost all diuretic medicinal plants and which are associated with their diurectic effect. However, the well-known surface activity of many of the saponines is considered to cause a number of undesirable adverse reactions encountered with the use of this group of medicinal plants and, thus, limits their field of application and the tolerable administrable dosage thereof. The latter, however, are critical factors for the therapeutic success. Particularly, the hemolytic, emetic and cytotoxic activities of the saponines limit the range of use of saponine-containing medicinal plants significantly. For example, commercial available extracts of Asparagus roots have a content of saponines in the range of 5 to 10 % (based on the weight of the dried extract).

Surprisingly, it turned out that it is possible, with the process of the present invention to provide Asparagus extracts which have an excellent diuretic and antihypertensive effect and which simultaneously are free or almost free of undesired saponines. It was particularly surprising that, at least for derivatives of Asparagus Officinalis L. roots and/or shoots, the diuretic effect observed is not at all associated with the presence of saponines, as was repeatedly stated in the prior art. Thus, it is possible, to provide a preparation, which exhibits excellent diuretic activity but with no or almost no adverse reactions, which are typically associated with the saponines. Therefore the Asparagus extracts of the present invention are suitable for the prevention and therapy of diseases, which cannot be medicated with common Asparagus preparations, due to their adverse reaction profile.

Since many years, synthetic diuretics and among them hydrochlorothiazide (HCT) as a standard substance widely used belong - beside Ca antagonists, β blocker, ACE inhibitors and angiotensin II antagonists - to the standard therapeutica for the treatment of high blood pressure (hypertension). These diuretics are used either alone or in combination with the above-mentioned antihypertensive agents.

Because of their marked aquarese efficiency via the kidneys, diuretics have an antihypertensive and cardio protective effect.

However, the diuretics of the prior art show side effects which are undesired. In particular, hydrochlorothiazide (HCT) secrets water by the excretion of sodium and potassium which leads to a massive loss of electrolytes (shift of the electrolyte balance) in the body.

This massive shift of the electrolyte balance may lead to significant side effects, for example arrhythmia, problems of sexual potency, diabetes etc..

The present invention aims to overcome the above described disadvantages.

Document Database WPI Section Ch, Week 198851 Derwent Pulbications Ltd., London, GB ; Class D13, AN 1988-363978 XP 002318108 refering to JP 63 273462 A (Marutomo KK), 10 November 1988 discloses the preparation of powdered tea from asparagus by cutting the stem of green asparagus, drying it, crushing it and roasting the crushed product. The powdered tea is extracted with hot water.

Document Hazebroek J.P. et al: "Allelopathic substances in asparagus roots extraction characterization and biological activity" in Journal of the American Society for horticultural Science, Vol. 114, No. 1, 1989, pages 152 to 158 teaches the crude extraction of Asparagus officinalis with water and further fractioning of the crude extract.

Document Hartung A.C. et al.: "Isolation and characterization of phytotoxic compounds from asparagus (asparagus officinalis L.) roots" in Journal of Chemical Ecology, Vol. 16, No. 5, 1990, pages 1707 - 1718 relates to the overnight extraction of Asparagus officinalis with water, its filtration and concentration to obtain a raw extract.

Document Shao Y. et al.: "Steroidal saponins from Asparagus officinalis and their cytotoxic activity" in Planta Medica, Vol. 63, No. 3, June 1997, pages 258 - 262 relates to the isolation and structural identification of two steroidal saponins from the methanol extract of asparagus seeds.

Document US 2003/181,395 discloses a process for the isolation of oligospiro-stanoside isolated from Asparagus racemosus with a polar solvent with or without prior extraction with EtOAc.

An object of the present invention is to provide saponine-free extracts having a potent effect as diuretic and antihypertensive drug, starting from a raw extract originating from roots and/or shoots of Asparagus Officinalis L. as an intermediate which not only has diuretic and antihypertensive effects itself, but also is a broadly available starting material for saponine-free extracts from roots and/or shoots of Asparagus Officinalis L..

It is a further object of the present invention is to provide a method for the preparation of saponine-free extracts from said raw-extract.

Another object of the present invention is to provide the use of said saponine-free extracts from Asparagus Officinalis L. roots and/or shoots for the promotion of a diuresis, for the promotion of purification of the body, for the promotion of body weight reduction, for the prevention and/or therapy of heart failure, for the prevention and/or therapy of hypertension and/or for the prevention and/or therapy of heart hypertrophia.

Finally, it is also an object of the present invention to provide a process for the promotion of a diuresis, for the promotion of purification of the body, for the promotion of body weight reduction, for the prevention and/or therapy of heart failure, for the prevention and/or therapy of hypertension and/or for the prevention and/or treatment of heart hypertrophia.

Hence, the invention relates to a saponine-free extract originating from roots and/or shoots of Asparagus Officinalis L., obtainable by at least the steps of
a)' grinding dry roots and/or shoots of Asparagus Officinalis L. to obtain a dry powder;
b)' adding to the dry powder so obtained a solvent selected from the group consisting of water, at least one organic liquid miscible with water and mixtures of water and at least one organic liquid miscible with water to obtain a suspension;
c)' agitating the suspension so obtained for a time > 10 h and at a temperature not exposing the constituents of said suspension to the risk of a biological degradation;
d)' separating the constituents of the suspension not soluble in said solvent from those constituents soluble in said solvent to obtain a soluble fraction dissolved in the solvent and an insoluble fraction; and
e)' removing the solvent from the soluble fraction to obtain a raw extract; and optionally
f)' grinding the raw extract obtained to prepare a raw extract of roots and/or shoots of Asparagus Officinalis L. in the form of a powder;

(i) adding at least one solvent selected from the group consisting of organic liquids miscible with water and mixtures of water and at least one organic liquid miscible with water, wherein the solvent has a lower hydrophilicity than the solvent used for the preparation of the raw extract, to the solution of soluble constituents of the raw extract obtained in step d) to obtain a solution or suspension;
(ii) agitating the solution or suspension so obtained for a time > 30 min and at a temperature not exposing the constituents of said suspension to the risk of a biological degradation;
(iii) separating the liquid phases in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases;
(iv) removing, from the liquid phase with the solvent of relatively high hydrophilicity, the solvent to obtain a solid residue; and
(v) drying and optionally grinding the solid residue to obtain a fractioned saponine-free extract from roots and/or shoots of Asparagus Officinalis L.

Preferred embodiments of the saponine free extract are claimed in subclaims 2 to 17.

The Asparagus Officinalis plant to which the present invention relates is the perennial green plant with small rhizomes to which the taxonomical classification
Class: Monocotyledonae,
Order: Liliales,
Family: Liliaceae,
Subfamily: Aparagoideae,
Genus: Asparagus,
Species: Officinalis,
applies.

As starting material for preparing the raw extract of the invention, roots or shoots of Asparagus Officinalis L. are used. As used in the present specification and in the claims, the term "roots" is meant to include those parts of the Asparagus Officinalis L. plant which are remaining underground and, during cultivation, are nourishing the plant. As used in the present specification and claims, the term "shoots" is meant to include those parts of the Asparagus Officinalis L. plant which are formed by transformed leaves of the plant and, hence, are growing above the soil resulting in green shoots (due to the exposure to the natural sunlight resulting into a photosynthesis of the shoots) or are growing in raised beds shielding the shoots from the sunlight so that a white colour of the shoots prevails.

In a first step providing the source material for the extracts of the invention, the roots and/or shoots of Asparagus are reduced in size to small pieces which are carefully dried and ground to obtain a dry powder of roots and/or shoots of Asparagus Officinalis L..

In a second step, the dry powder obtained in the first step is subjected to a solvent extraction. The extraction is conducted by using one or more than one solvent. The use of one solvent is preferred. There may be used any solvent compatible with the final use, particularly the medical field use, of the extracts obtained. Useful solvents are selected from the group consisting of water, at least one organic liquid miscible with water and mixtures of water and at least one organic liquid miscible with water to obtain a suspension.

In a preferred embodiment of the invention, water is used as the solvent. Advantageously, when using water as the solvent, particularly saponine-free raw extracts are obtainable from roots and/or shoots of Asparagus Officinalis L.. When practically obtaining the preferred raw extracts of roots and/or shoots of Asparagus Officinalis L. of the present invention, water is used as the solvent in an amount of from 0,1 to 10 l of water per 100 g of the dried powder of Asparagus roots and/or shoots, more preferably in an amount of from 0,5 to 5 l of water per 100 g of the dried powder of Asparagus roots and/or shoots.

Alternatively, in another preferred embodiment of the invention, at least one organic liquid miscible with water and selected from the group consisting of straight-chain or branched or cyclic alcohols having 1 to 6 carbon atoms and straight-chain or branched or cyclic ketones having 3 to 6 carbon atoms may be used as the solvent. Preferably raw extracts of Asparagus Officinalis L. roots and/or shoots are obtainable when using one or more solvent(s) selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms. Even more preferable are raw extracts from Asparagus Officinalis roots and/or shoots obtainable by using a solvent selected from methanol and ethanol and acetone and mixtures thereof. Most preferred are raw extracts obtainable by using methanol and/or ethanol and/or acetone in an amount of from 0.5 to 5 of the solvent per 100 g of the dry powder of Asparagus Officinalis L. roots and/or shoots, utmost preferred in an amount of from 0.5 to 5 l of methanol and/or ethanol and/or acetone per 100 g of the dry powder of Asparagus Officinalis L. roots and/or shoots.

In a particularly preferred embodiment, as the solvent, a mixture of water and at least one organic liquid miscible with water and selected from the group consisting of straight-chain or branched or cyclic alcohols having 1 to 6 carbon atoms and straight-chain or branched or cyclic ketones having 3 to 6 carbon atoms may be used, preferably a mixture of water and an organic liquid selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms, even more preferable a mixture of water and an organic liquid selected from methanol and ethanol and acetone and mixtures thereof, even more preferable a mixture of water and up to 50 vol.-% of methanol and/or ethanol and/or acetone, even more preferred a mixture of water and 20 to 40 vol.-% of methanol and/or ethanol and/or acetone, even more preferred a mixture of water and 25 to 35 vol.-% of methanol and/or ethanol and/or acetone, most preferred said solvent in an amount of from 0.5 to 5 I of the solvent per 100 g of the dry powder of Asparagus Officinalis L. roots and/or shoots, utmost preferred said solvent in an amount of from 0.5 to 5 l of methanol and/or ethanol and/or acetone per 100 g of the dry powder of Asparagus Officinalis L. roots and/or shoots.

In a most preferred and particularly advantageous embodiment of the invention, the powdered Asparagus roots and/or shoots are extracted with either water or a mixture of water and 30 vol.-% ethanol so as to obtain a particularly advantageous raw extract.

In accordance with the invention, the extraction is carried out by agitating the asparagus/solvent suspension for a time > 10 h and at a temperature not exposing the constituents of said suspension to the risk of a biological degradation. Particularly preferred raw extracts from Asparagus Officinalis roots and/or shoots are obtainable by shaking said suspension, more preferably said aqueous or aqueous/alcoholic suspension, for a time in the range of 10 to 48 h and/or by shaking said suspension at a temperature in the range of 10 to 25 °C.

After the extraction step is completed, the constituents of the suspension not soluble in said solvent are separated from those constituents soluble in said solvent by any conceivably separation process suitable for separating liquid and solid materials. A person skilled in the art knows such separation processes and may select suitable separation steps without difficulties. Preferred raw extracts according to the invention are obtainable by means of processes selected from the group consisting of centrifugation, decantation, filtration and combinations thereof, thus obtaining a soluble fraction dissolved in the solvent and an insoluble fraction. The insoluble fraction may again be rinsed with said solvent and the soluble fractions are combined whereas the solid residue is discarded.

After the separation step, the solvent of the soluble fraction is removed. The solvent removal can be conducted by any process suitable for carefully removing solvents from solutions without taking risks of a chemical degradation of any of the components desired to be obtained. Preferred raw extracts from Asparagus Officinalis L. roots and/or shoots are obtainable either by at least one step selected from the group consisting of evaporating the solvent partly and crystallizing the solute, evaporating the solvent completely and recovering the dry solute, freeze drying the solution and combinations of those steps. As a result, a saponine-depleted raw extract of roots and/or shoots of asparagus may be obtained in good yields and having surprisingly low saponine contents.

In an optional step, the dry raw extract obtained is ground, resulting into a dry powder of an Asparagus Officinalis L. roots and/or shoots raw extract.

In the first step of preparing the saponine-free extract, at least one solvent selected from the group consisting of organic liquids miscible with water and mixtures of water and at least one organic liquid miscible with water, wherein the solvent has a lower hydrophilicity than the solvent used for the preparation of the raw extract is added as the extraction solvent to the said raw extract or to the powdered raw extract to obtain a solution or suspension.

In an alternative solution for preparing the saponine-free extract the afore-mentioned solvent for the extraction is added to a solution of the raw extract which is selected from the group consisting of water or the solvent which was used for obtaining the raw extract, to obtain a solution or suspension, wherein the solvent has a lower hydrophilicity than the solvent used for the preparation of the raw extract.

In a still further alternative, the extraction may also be carried out by adding the afore-mentioned extraction solvent directly to the solution of soluble constitutents in the process for preparing the raw extract to obtain a solution or suspension, wherein the solvent has a lower hydrophilicity than the solvent used for the preparation of the raw extract. For example, the extraction may be carried out by liquid-liquid extraction (extraction in a liquid-liquid phase).

The solvent used in the above extraction-step is a solvent comprising a mixture of water and a high content of at least one organic liquid miscible with water, preferably a solvent comprising water and a high content of at least one organic liquid selected from the group consisting of straight-chain or branched or cyclic alcohols having 1 to 6 carbon atoms and straight-chain or branched or cyclic ketones having 3 to 6 carbon atoms, more preferably a solvent comprising water and a high content of an organic liquid selected from the group consisting of straight-chain or branched aliphatic alcohols having 1 to 3 carbon atoms and straight-chain or branched aliphatic ketones having 3 to 5 carbon atoms, even more preferably a solvent comprising water and a high content of methanol and/or ethanol and/or acetone, even more preferred a solvent comprising water and more than 50 vol.-% of methanol and/or ethanol and/or acetone, even more preferred a solvent comprising water and 60 to 90 vol.-% of methanol and/or ethanol and/or acetone, most preferred a solvent comprising water and 75 to 95 vol.-% of methanol and/or ethanol and/or acetone or by using, as a solvent, a purely organic liquid, preferably an organic liquid selected from the group consisting of straight-chain or branched or cyclic alcohols having 1 to 6 carbon atoms and straight-chain or branched or cyclic ketones having 3 to 6 carbon atoms, more preferably an organic liquid selected from the group consisting of straight-chain or branched aliphatic alcohols having 1 to 3 carbon atoms and straight-chain or branched aliphatic ketones having 3 to 5 carbon atoms, even more preferably an organic liquid selected from the group consisting of methanol and/or ethanol and/or acetone.

In a most preferred embodiment, the solvent for the extraction is ethanol.

For an effective extraction, the solution or suspension from the preceding step is agitated. Any agitation process may be applied for obtaining excellent saponine-free Asparagus Officinalis L. roots and/or shoots extracts. In preferred embodiments of the invention, the saponine-free extracts of the invention are obtainable for example by shaking the suspension or solution of the previous step, preferably by shaking the suspension or solution of the previous step for a time in the range of from 30 to 90 min and/or shaking the suspension or solution at a temperature in the range of from 10 to 25 °C.

After the completion of the extraction-step, the liquid phase is separated from any insoluble constituent to obtain a solid residue which is insoluble in the extraction solvent of the liquid phase. Any suitable separation process may be used, without that the invention is restricted to a specific separation step. Examples of the separation step are steps of filtration, decantation, centrifugation or flotation or combinations of such separation processes.

In order to separate insoluble and soluble constituents the suspension is preferably subjected to at least one step selected from the group consisting of centrifugation, decantation, filtration and combinations of those steps so as to obtain a liquid phase and a solid residue.

Finally, the solid phase from the separation step is dried and optionally ground to obtain a fractioned saponine-free extract from roots and/or shoots of Asparagus Officinalis L..

With regard to the further described alternative embodiments, in both cases the liquid phases are separated from each other in accordance with a suitable parameter thereof and are, optionally, separated from any insoluble constituent to obtain a liquid phase with a solvent of relatively high hydrophilicity and a liquid phase with a solvent of relatively lower hydrophilicity and, optionally, a solid phase insoluble in both liquid phases.

Subsequently, the solid residue is recovered from the liquid phase with the solvent of relatively high hydrophilicity by removing the solvent, drying the obtained solid residue and optionally grinding. The product is a fractioned saponine-free extract from roots and/or shoots of Asparagus Officinalis L..

The removal of the solvent from the solution or suspension may be carried out by means of evaporating the solvent partly and crystallizing the solute, evaporating the solvent completely and recovering the dry solute, freeze drying the solution and combinations of those steps. However, the invention is not at all restricted to those steps of removing the solvent so as to obtain the solute.

The saponine-free extracts are, in particularly advantageous embodiments of the invention, prepared from an aqueous raw extract from roots or shoots of Asparagus Officinalis L or from a hydro-alcoholic, preferably from a hydroethanolic, more preferably from a hydro-(20- to 40-vol.-% ethanolic) raw extract from roots or shoots of Asparagus Officinalis L.

In a preferred embodiment of the present invention, the saponine-free extract is prepared using an aqueous raw extract from roots or shoots of Asparagus Officinalis L.

The raw extracts from roots and/or shoots of Asparagus Officinalis L. have a saponine content of < 5 % by weight, preferably of 2 to 3 % by weight, even more preferred of < 2 % by weight.

The fractioned saponine-free extracts from roots and/or shoots of Asparagus Officinalis L. are substantially saponine-free.

In addition, the present invention concerns a process for preparing a saponine-free extract from roots and/or shoots of Asparagus Officinalis, comprising the steps of
a) grinding dry roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and optionally
f) grinding the raw extract obtained to prepare a saponine-depleted raw extract of roots and/or shoots of Asparagus Officinalis L. in the form of a powder;

(i) adding at least one solvent selected from the group consisting of organic liquids miscible with water and mixtures of water and at least one organic liquid miscible with water, wherein the solvent has a lower hydrophilicity than the solvent used for the preparation of the raw extract, to the solution of soluble constituents of the raw extract obtained in step d) to obtain a solution or suspension;
(ii) agitating the solution or suspension so obtained for a time > 30 min and at a temperature not exposing the constituents of said suspension to the risk of a biological degradation;
(iii) separating the liquid phases in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases;
(iv) removing, from the liquid phase with the solvent of relatively high hydrophilicity, the solvent to obtain a solid residue; and
(v) drying and optionally grinding the solid residue to obtain a fractioned saponine-free extract from roots and/or shoots of Asparagus Officinalis L..

Preferred embodiments of said process are claimed in subclaims 19 to 36.

As starting material for preparing the raw extract roots or shoots of Asparagus Officinalis L. are used.

In a first step of the process the roots and/or shoots of Asparagus are reduced to small pieces, dried and ground to obtain a dry powder.

In a second step, the powder is extracted by using different solvents, which are selected from the group consisting of water, at least one organic liquid miscible with water and mixtures of water and at least one organic liquid miscible with water to obtain a suspension. In a preferred embodiment water is used as the solvent in an amount of from 0,1 to 10 I of water per 100 g of the dried powder of asparagus roots and/or shoots, more preferably in an amount of from 0,5 to 5 1 of water per 100 g of the dried powder of asparagus roots and/or shoots.

Alternatively, as the solvent, at least one organic liquid miscible with water and selected from the group consisting of straight-chain or branched or cyclic alcohols having 1 to 6 carbon atoms and straight-chain or branched or cyclic ketones having 3 to 6 carbon atoms, preferably selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms, even more preferable selected from methanol and ethanol and acetone and mixtures thereof, most preferred in an amount of from 0.5 to 5 I of the solvent per 100 g of the dry powder of Asparagus Officinalis L. roots and/or shoots, utmost preferred in an amount of from 0.5 to 5 I of methanol and/or ethanol and/or acetone per 100 g of the dry powder of Asparagus Officinalis L. roots and/or shoots may be used.

In a preferred embodiment, as the solvent, a mixture of water and at least one organic liquid miscible with water and selected from the group consisting of straight-chain or branched or cyclic alcohols having 1 to 6 carbon atoms and straight-chain or branched or cyclic ketones having 3 to 6 carbon atoms, preferably a mixture of water and an organic liquid selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms, even more preferable a mixture of water and an organic liquid selected from methanol and ethanol and acetone and mixtures thereof, even more preferable a mixture of water and up to 50 vol.-% of methanol and/or ethanol and/or acetone, even more preferred a mixture of water and 20 to 40 vol.-% of methanol and/or ethanol and/or acetone, even more preferred a mixture of water and 25 to 35 vol.-% of methanol and/or ethanol and/or acetone, most preferred said solvent in an amount of from 0.5 to 5 l of the solvent per 100 g of the dry powder of Asparagus Officinalis L. roots and/or shoots, utmost preferred said solvent in an amount of from 0.5 to 5 l of methanol and/or ethanol and/or acetone per 100 g of the dry powder of Asparagus Officinalis L. roots and/or shoots may be used.

In a most preferred embodiment, the powdered Asparagus roots and/or shoots are extracted with either water or a mixture of water and 30 vol.-% ethanol.

The extraction is carried out by agitating the asparagus/solvent suspension for a time > 10 h and at a temperature not exposing the constituents of said suspension to the risk of a biological degradation, preferably by shaking said suspension for a time in the range of 10 to 48 h and/or shaking said suspension at a temperature in the range of 10 to 25 °C.

After the extraction step is completed, the constituents of the suspension not soluble in said solvent are separated from those constituents soluble in said solvent by means of centrifugation, decantation, filtration and combinations thereof and thus obtaining a soluble fraction dissolved in the solvent and an insoluble fraction. The insoluble faction is again rinsed with said solvent and the solvent fractions are combined whereas the solid residue is discarded.

After the separation step, the solvent of the soluble fraction is removed either by at least one step selected from the group consisting of evaporating the solvent partly and crystallizing the solute, evaporating the solvent completely and recovering the dry solute, freeze drying the solution and combinations of those steps to prepare a saponine-depleted raw extract of roots and/or shoots of asparagus.

In an optionally step the dry raw extract obtained is ground.

In the first step of preparing the saponine-free extract, at least one solvent selected from the group consisting of organic liquids miscible with water and mixtures of water and at least one organic liquid miscible with water, wherein the solvent has a lower hydrophilicity than the solvent used for the preparation of the raw extract is added to the raw extract or to the powdered raw extract to obtain a solution or suspension.

In an alternative solution for preparing the saponine-free extract the afore-going solvent for the extraction is added to a solution of the raw extract which is selected from the group consisting of water or the solvent which was used for obtaining the raw extract, to obtain a solution or suspension.

In a still further alternative the extraction may also be carried out by adding the afore-going solvent directly to the solution of soluble constituents in the process for preparing the raw extract to obtain a solution or suspension. For example, the extraction may be carried out by liquid-liquid extraction (extraction on a liquid-liquid phase).

The solvent used in the above extraction-step is a solvent comprising a mixture of water and a high content of at least one organic liquid miscible with water, preferably a solvent comprising water and a high content of at least one organic liquid selected from the group consisting of straight-chain or branched or cyclic alcohols having 1 to 6 carbon atoms and straight-chain or branched or cyclic ketones having 3 to 6 carbon atoms, more preferably a solvent comprising water and a high content of an organic liquid selected from the group consisting of straight-chain or branched aliphatic alcohols having 1 to 3 carbon atoms and straight-chain or branched aliphatic ketones having 3 to 5 carbon atoms, even more preferably a solvent comprising water and a high content of methanol and/or ethanol and/or acetone, even more preferred a solvent comprising water and more than 50 vol.-% of methanol and/or ethanol and/or acetone, even more preferred a solvent comprising water and 60 to 90 vol.-% of methanol and/or ethanol and/or acetone, most preferred a solvent comprising water and 75 to 95 vol.-% of methanol and/or ethanol and/or acetone or by using, as a solvent, a purely organic liquid, preferably an organic liquid selected from the group consisting of straight-chain or branched or cyclic alcohols having 1 to 6 carbon atoms and straight-chain or branched or cyclic ketones having 3 to 6 carbon atoms, more preferably an organic liquid selected from the group consisting of straight-chain or branched aliphatic alcohols having 1 to 3 carbon atoms and straight-chain or branched aliphatic ketones having 3 to 5 carbon atoms, even more preferably an organic liquid selected from the group consisting of methanol and/or ethanol and/or acetone.

In a most preferred embodiment, the solvent for the extraction is ethanol.

For an effective extraction, the solution or suspension from the preceding step is agitated, for example by shaking the suspension or solution, preferably by shaking the suspension or solution for a time in the range of from 30 to 90 min and/or shaking the suspension or solution at a temperature in the range of from 10 to 25 °C.

After the completion of the extraction-step, the liquid phase is separated from any insoluble constituent to obtain a solid residue which is insoluble in the liquid phase. In order to separate insoluble and soluble constituents the suspension is subjected at least one step selected from the group consisting of centrifugation, decantation, filtration and combinations of those steps obtaining a liquid phase and a solid residue.

Finally, the solid phase from the separation step is dried and optionally ground to obtain a fractioned saponine-free extract from roots and/or shoots of Asparagus Officinalis L..

With regard to the further described alternative embodiments in both cases the liquid phases are separated in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases.

Subsequently, the solid residue is recovered from the liquid phase with the solvent of relatively high hydrophilicity by removing the solvent, drying the obtained solid residue and optionally grinding. The product is a fractioned saponine-free extract from roots and/or shoots of Asparagus Officinalis L.

The removal of the solvent from the solution or suspension may be carried out by means of evaporating the solvent partly and crystallizing the solute, evaporating the solvent completely and recovering the dry solute, freeze drying the solution and combinations of those steps.

The saponine-free extracts are prepared from an aqueous raw extract from roots or shoots of Asparagus Officinalis L or from a hydro-alcoholic, preferably from a hydroethanolic, more preferably from a hydro-(20- to 40-vol.-% ethonolic) raw extract from roots or shoots of Asparagus Officinalis L.

In a preferred embodiment of the present invention, the saponine-free extract is prepared using an aqueous raw extract from roots or shoots of Asparagus Officinalis L.

Furthermore, the present invention relates to a pharmaceutical preparation or nutritional supplement, in particular a vegetable diuretic preparation comprising one or more of saponine-free extracts from Asparagus Officinalis L. roots and/or shoots as defined above.

These preparations may optionally comprise one or more phamacologically acceptable carrier substances, auxiliary substances and/or vehicles. Furthermore, the preparations may further, comprise vegetable extracts which are selected from broccoli, sweet corn, stinging-nettle, parsley, pineapple, pawpaw, mango etc. which have an additive diuretic effect. In addition, vitamins like vitamin A, vitamin B, vitamin C, vitamin E, biotin, mineral nutrients, trace elements and/or natural flavouring substances may be added to the preparation.

In a most preferred embodiment, the pharmaceutical preparation or nutritional supplement, in particular vegetable diuretic preparation comprises the saponine-free ethanol-insoluble component of the aqueous or of the hydro-alcoholic raw extract of Asparagus Officinalis L. roots and/or shoots.

Moreover, the present invention relates to the use of one or more of the components of saponine-free extracts from Asparagus Officinalis L. roots and/or shoots as defined above for the manufacture of a medicament for the promotion of a diuresis, for the promotion of purification of the body, for the promotion of body weight reduction, for the prevention and/or treatment of heart failure, for the prevention and/or treatment of hypertension and/or for the prevention and/or treatment of heart hypertrophia.

In addition, the present invention relates to a process for the promotion of a diuresis, for the promotion of purification of the body, for the promotion of body weight reduction, for the prevention and/or treatment of heart failure, for the prevention and/or treatment of hypertension and/or for the prevention and/or treatment of heart hypertrophia in a mammal, preferably in a human, comprising an administration of one or more of the components of saponine-free extracts from Asparagus Officinalis L. roots and/or shoots as described in detail above to a mammal, preferably to a human, in need of such a promotion of conditions and/or prevention and/or treatment of diseases in an amount affording an effective promotion of conditions and/or prevention and/or treatment of diseases.

### Brief description of the drawings

- Figure 1 is a flow sheet showing the steps for the preparation of the raw extract.
- Figure 2 is a flow sheet showing the steps for the extraction for preparing the saponine-free fractioned extract.
- Figure 3 is a diagram showing the cumulative mean volume of urine depending on the time for extracts "roots".
- Figure 4 is a diagram showing the cumulative mean volume of urine depending on the time for extracts "shoots".
- Figure 5 is diagram showing the cumulative mean volume of urine excreted by rats depending on the time for fractioned aqueous extracts of "shoots".

The present invention will now be described in more detail by the following Examples. It is intended that the Examples explain, but do not restrict the invention.

### Example 1

### Preparation of different extracts of Asparagus Officinalis L.

### Vegetable Extracts

The extracts used were prepared form roots and shoots of Asparagus Officinalis L., independently.

### Extracts prepared from parts of roots:

### → aqueous raw extract: Preparation Example 1

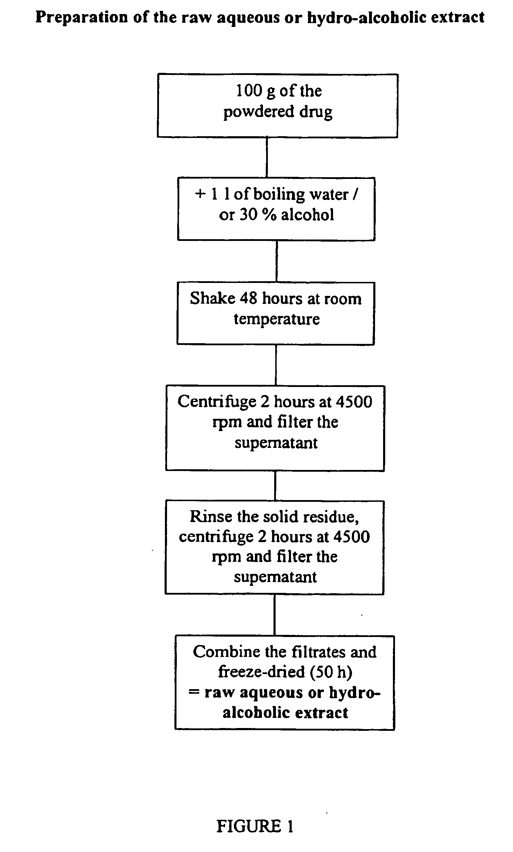

100 g of the Asparagus Officinalis L. roots in the form of a powder dried carefully under controlled conditions were suspended in 1.000 ml of water. The suspension was shaken at 20 °C (room temperature) for 48 h. The thus obtained mixed suspension was subjected to centrifugation at 4.500 rpm for 2 hours. The solid residue was rinsed with water and again subjected to a centrifugation step at 4.500 rpm for 2 hours. The solid residue resulting from said second centrifugation step was discarded. The supernatants of the two centrifugation steps were combined, and the water was removed by freeze-drying. There was obtained a dark-brown solid in a yield of 11 % by weight, based on the weight of the starting roots powder. The sodium content was 125 mg and the potassium content was 121 mg (based on 100 g of dried plant starting material). This process is shown in the scheme of Figure 1.

### → hydro-alcoholic raw extract: Preparation Example 2

The above procedure (of obtaining the aqueous raw extract; preparation example 1) was repeated, with the exception that a mixture of 30 vol.-% ethanol and 70 vol.-% water was used for the extraction. The product was a light-brown solid in a yield of 10 % by weight of the starting roots powder. The sodium content was 126 mg and the potassium content was 106 mg (based on 100 g of dried plant starting material). This process is shown in the scheme of Figure 1.

### → fractioned aqueous extract: Preparation Example 3

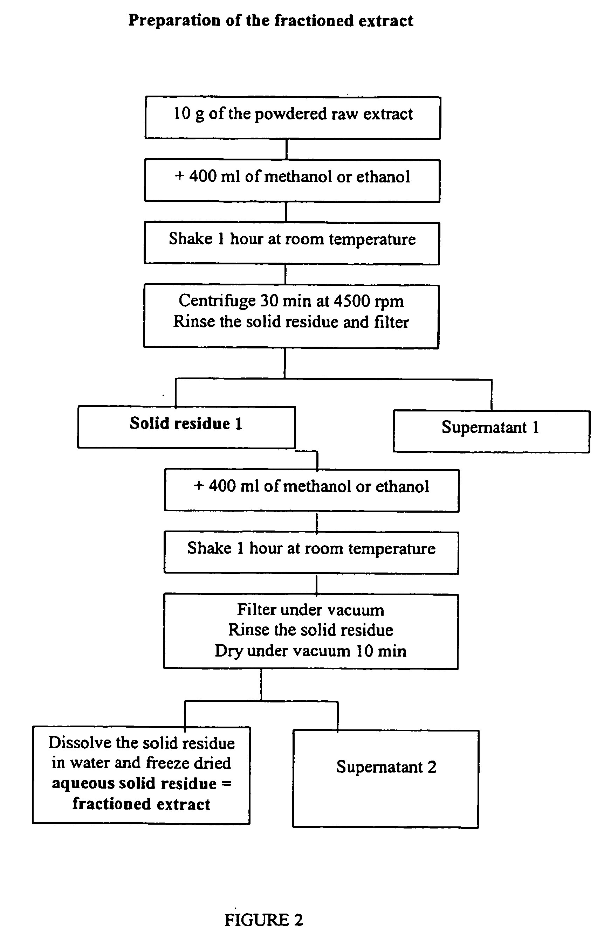

The dry powder of the aqueous raw extract of preparation example 1 (10 g) was suspended in 400 ml ethanol. The suspension was shaken for 60 min at 20 °C (ambient temperature). The suspension thus obtained was subjected to centrifugation at 4.500 rpm for 30 minutes. The solid residue was rinsed with 400 ml ethanol, subjected to filtration and dried under vacuum. The solid residue resulting from said drying step was recovered; yield: 56 % by weight, based on the amount of raw extract employed. The sodium content was 90 mg and the potassium content was 78 mg (based on 100 g of dried plant starting material). The contents of the supernatant of the centrifugation and filtration steps were not further considered for the further study. This process is shown in the scheme of Figure 2.

### → fractioned hydro-alcoholic extract: Preparation Example 4

The dry powder of the hydro-alcoholic raw extract of preparation example 2 (10 g) was suspended in 400 ml ethanol. The suspension was shaken for 60 min at 20 °C (ambient temperature). The suspension thus obtained was subjected to centrifugation at 4.500 rpm for 30 minutes. The solid residue was rinsed with 400 ml ethanol, subjected to filtration and dried under vacuum. The solid residue resulting from said drying step was recovered; yield: 47 % by weight, based on the amount of raw extract employed. The sodium content was 35 mg and the potassium content was 19 mg (based on 100 g of dried plant starting material). The contents of the supernatant of the centrifugation and filtration steps were not further considered for the further study. This process is shown in the scheme of Figure 2.

### Extracts prepared from parts of shoots:

### → aqueous raw extract: Preparation Example 5

100 g of the Asparagus Officinalis L. shoots in the form of a powder dried carefully under controlled conditions were suspended in 1.000 ml of water. The suspension was shaken at 20 °C (room temperature) for 48 h. The thus obtained mixed suspension was subjected to centrifugation at 4.500 rpm for 2 hours. The solid residue was rinsed with water and again subjected to a centrifugation step at 4.500 rpm for 2 hours. The solid residue resulting from said second centrifugation step was discarded. The supernatants of the two centrifugation steps were combined, and the water was removed by freeze-drying. There was obtained a light-brown solid in a yield of 31 % by weight, based on the weight of the starting shoots powder. The sodium content was 198 mg and the potassium content was 1,954 mg (based on 100 g of dried plant starting material). This process is shown in the scheme of Figure 1.

### → hydro-alcoholic raw extract: Preparation Example 6

The above procedure (of obtaining the aqueous raw extract; preparation example 5) was repeated, with the exception that a mixture of 30 vol.-% ethanol and 70 vol.-% water was used for the extraction. The product was a light-brown solid in a yield of 33 % by weight of the starting roots powder. The sodium content was 210 mg and the potassium content was 2,210 mg (based on 100 g of dried plant starting material). This process is shown in the scheme of Figure 1.

### → fractioned aqueous extract: Preparation Example 7

The dry powder of the aqueous raw extract of preparation example 5 (10 g) was suspended in 400 ml ethanol. The suspension was shaken for 60 min at 20 °C (ambient temperature). The suspension thus obtained was subjected to centrifugation at 4.500 rpm for 30 minutes. The solid residue was rinsed with 400 ml ethanolsubjected to filtration and dried under vacuum. The solid residue resulting from said drying step was recovered. The contents of the supernatant of the centrifugation and filtration steps were not further considered for the further study. This process is shown in the scheme of Figure 2.

### → fractioned hydro-alcoholic extract: Preparation Example 8

The dry powder of the hydro-alcoholic raw extract of preparation example 6 (10 g) was suspended in 400 ml ethanol. The suspension was shaken for 60 min at 20 °C (ambient temperature). The suspension thus obtained was subjected to centrifugation at 4.500 rpm for 30 minutes. The solid residue was rinsed with ethanol, subjected to filtration and dried under vacuum. The solid residue resulting from said drying step was recovered. The contents of the supernatant of the centrifugation and filtration steps were not further considered for the further study. This process is shown in the scheme of Figure 2.

The saponine contents of the starting materials (powders of roots or shoots of Asparagus Officinalis L.), raw extracts and fractioned extracts of roots or shoots of Asparagus Officinalis L. were determined by thin layer chromatography on silica gel. The results revealed a relatively high saponine content of the starting materials (as expected). In the solid residues of the ethanolic fractionation steps (preparation examples 3, 4, 7 and 8), the thin layer chromatography showed substantially saponine-free products.

### Example 2

### Diuretic and antihypertensive effects of different extracts of Asparagus Officinalis L.

### Animals

The animals employed in the tests were male OFA Sprague-Dawley rats (obtained from the Centre d'élevage Iffa-Credo), each having a weight in the range of from 225 to 250 g. All the animals later employed in the tests were held in individual cages at a temperature of 21 ± 1 °C and were subjected to a day/night cycle of 12/12 h for 8 days to adapt them to the test environment. During this phase, the animals were fed a standardized feed (croquettes M 20; SDS, G.B.) and water *ad libitum.*

### Experimental protocol

### • Adaptation

48 h before the start of the experiment the rats were placed in a cage allowing separate feeding and individual metabolism of each of the animals. Feed and drink (water) *ad libitum.*

### • Experimentation

After the above 48 h period, each animal was administered an amount of 5 ml of an aqueous NaCl solution (0.45 % NaCl) per 100 g body weight of the animal on the intraperitoneal administration route, to which solution was added the product to be tested in the case of the treated animal group. In the cases of animals of the control group, only the saline solution was administered intraperitoneally. The animals were held under diet for 8 h after the administration in their metabolism cages. The urine of all animals was collected in graduated polypropylene vials, and their volumes were noted every hour during the 8 h period. After 8 h, the vials were removed from the animals, the urine pH value was measured (portable pH meter: PHM 201 Meterlab^{R}; Choffel, France), and the contents of K⁺ and Na⁺ were measured by emission spectrometry (Spectraspan VII; Spectrametrics).

### • Tests

→ Control group: NaCl 0,45 % (n = 10 rats)
→ Reference group: hydrochlorothiazide (Esidrix^{R}; Ciba-Geigy) 10 mg/kg weight (n = 10 rats); hydrochlorothiazide is used as standard diuretic.
→ Vegetable extract "roots" group: each extract was tested on the basis of 400 mg starting material (dried plant material)/kg weight;
   The number of animals per group was 10 for the aqueous (preparation example 1) and hydro-alcoholic (preparation example 2) raw extracts and 12 animals each for the fractioned aqueous (preparation example 3) and fractioned hydro-alcoholic (preparation example 4) extracts;
→ Vegetable extraxt "shoots" group: each raw extract was tested in two different concentrations, namely 400 mg and 116 mg dried plant/kg weight for the aqueous raw extract (preparation example 5) and 400 mg and 109 mg dried plant/kg weight for the hydro-alcoholic raw extract (preparation example 6);
   The number of animals per group was 10 in each case.

### (4) Diuretic effects:

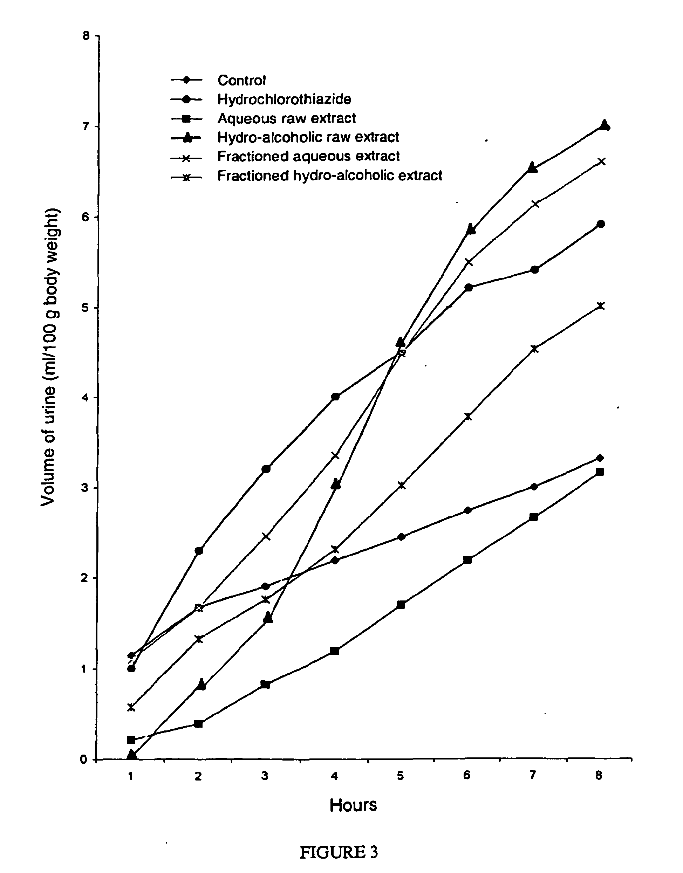

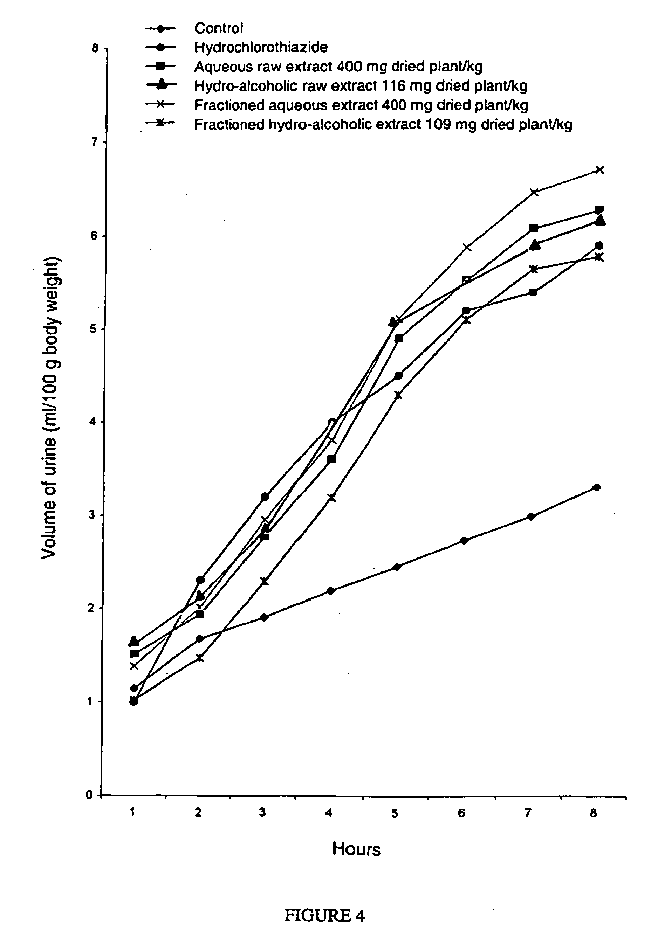

The diuretic effect of the extracts of preparation examples 1 to 4 and of the extracts of preparation examples 5 and 6 are shown in Figures 3 and 4, respectively.

It is obvious from Figure 3 that, with respect to extracts originating from Asparagus roots, the hydro-alcoholic raw extract (product of preparation example 2), the fractioned aqueous extract (product of preparation example 3), and the fractioned hydro-alcoholic extract (product of preparation example 4) had excellent diuretic effects comparable to or even better than hydrochlorothiazide which is a potent diuretic of the prior art, if administered in the above concentrations.

As shown in Figure 4, with respect to extracts originating from Asparagus shoots, the aqueous raw extract (product of preparation example 5) and the hydro-alcoholic raw extract (product of preparation example 6) in an administration dose of 400 mg dried plant/kg body weight showed considerably better diuretic effects than the reference substance hydrochlorothiazide (standard diuretic). The hydro-alcoholic raw extract (product of preparation example 6) if administered in an amount of 109 mg dried plant/kg body weight showed a diuretic effect similar to the effect exerted by the reference substance hydrochlorothiazide.

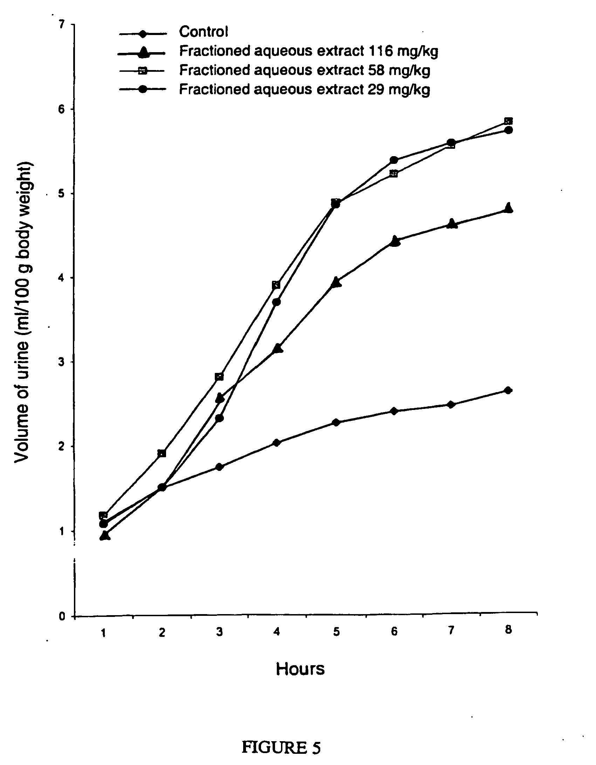

A surprising result was found with respect to the fractioned aqueous extracts of Asparagus shoots (preparation example 7): If administered in (a) an amount of 116 mg (basis: dried plant starting material) per kg body weight; (b) an amount of 58 mg (basis: dried plant starting material) per kg body weight; and (c) an amount of 29 mg (basis: dried plant starting material) per kg body weight, the latter two administrations exert considerably better results than the former one (a). This is shown in Figure 5. In other words: Lower concentrations of the di-uretically effective extracts, administered under comparable conditions, result into better effects than the higher concentration of the vegetable diuretic.

### (5) Evaluation of the results:

To summarise the above results it can be stated that the extracts of the present invention exhibit excellent diuretic effects compared to the standard diuretic substance hydrochlorothiazide (HCT).

Furthermore, the diuretic effect - as known in the art - is accompanied by an antihypertensive effect not only in combination with other hypertensive drugs but also when administered alone.

With the extracts of the present invention the disadvantageous side effects caused by the simultaneously excretion of sodium and potassium ions can be diminished: The extracts of the present invention contain sodium and potassium in considerable amounts. Due to the alkali metal ion content (see preparation examples) the administration of the inventive extracts compensates the diuretic excretion of sodium and potassium ions widely. The improvement of the electrolyte balance overcomes the disadvantages of the prior art diuretics.

## Claims

1. A saponine-free extract from roots and/or shoots of Asparagus Officinalis L., obtainable by at least the steps of
a) grinding dry, roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and
f) optionally grinding the raw extract obtained to prepare a raw extract of roots and/or shoots of Asparagus Officinalis L. in the form of a powder;
(i) adding at least one solvent selected from the group consisting of organic liquids miscible with water and mixtures of water and at least one organic liquid miscible with water, wherein the solvent has a lower hydrophilicity than the solvent used for the preparation of the raw extract, to the solution of soluble constituents of the raw extract obtained in step d) to obtain a solution or suspension;
(ii) agitating the solution or suspension so obtained for a time > 30 min and at a temperature not exposing the constituents of said suspension to the risk of a biological degradation;
(iii) separating the liquid phases in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases;
(iv) removing, from the liquid phase with the solvent of relatively high hydrophilicity, the solvent to obtain a solid residue; and
(v) drying and optionally grinding the solid residue to obtain a fractioned saponine-free extract from roots and/or shoots of Asparagus Officinalis L..

2. A saponine-free extract from roots and/or shoots of Asparagus Officinalis L., obtainable by at least the steps of
a) grinding dry roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and
f) optionally grinding the raw extract obtained to prepare a raw extract of roots and/or shoots of Asparagus Officinalis L. in the form of a powder;
(i) adding at least one solvent selected from the group consisting of organic liquids miscible with water and mixtures of water and at least one organic liquid miscible with water, wherein the solvent has a lower hydrophilicity than the solvent used for the preparation of the raw extract, to a solution of the raw extract obtained in step e) and/or step f) in a solvent selected from the group of water and the solvent which was used for obtaining the raw extract in steps a) to d), to obtain a solution or suspension;
(ii) agitating the solution or suspension so obtained for a time > 30 min and at a temperature not exposing the constituents of said suspension to the risk of a biological degradation;
(iii) separating the liquid phases in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases;
(iv) removing, from the liquid phase with the solvent of relatively high hydrophilicity, the solvent to obtain a solid residue; and
(v) drying and optionally grinding the solid residue to obtain a fractioned saponine-free extract from roots and/or shoots of Asparagus Officinalis L..

3. A saponine-free extract from roots and/or shoots of Asparagus Officinalis L., obtainable by at least the steps of
a) grinding dry roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and
f) optionally grinding the raw extract obtained to prepare a raw extract of roots and/or shoots of Asparagus Officinalis L. in the form of a powder;
(i) adding at least one solvent selected from the group consisting of organic liquids miscible with water and mixtures of water and at least one organic liquid miscible with water, wherein the solvent has a lower hydrophilicity than the solvent used for the preparation of the raw extract, to the raw extract obtained in step e) and/or f) to obtain a suspension;
(ii) agitating the suspension so obtained for a time> 30 min and at a temperature not exposing the constituents of said suspension to the risk of a biological degradation;
(iii) separating the liquid phase from any insoluble constituent to obtain a solid residue insoluble in the liquid phase; and
(iv) drying and optionally grinding the solid residue to obtain a fractioned saponine-free extract from roots and/or shoots of Asparagus Officinalis L..

4. The saponine-free extract of any of the claims 1 to 3, wherein in step b) water is used as the solvent, preferably water as the solvent in an amount of from 0.1 to 10 l of water per 100 g of a dry powder of Asparagus Officinalis L. roots and/or shoots, more preferred water as the solvent in an amount of from 0.5 to 5 I of water per 100 g of a dry powder of Asparagus Officinalis L. roots and/or shoots.

5. The saponine-free extract of any of the claims 1 to 3, wherein in step b) there is used as the solvent at least one organic liquid miscible with water and selected from the group consisting of straight-chain or branched or cyclic alcohols having 1 to 6 carbon atoms and straight-chain or branched or cyclic ketones having 3 to 6 carbon atoms, preferably selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms, even more preferable selected from methanol and ethanol and acetone and mixtures thereof, most preferred in an amount of from 0.5 to 5 l of the solvent per 100 g of the dry powder of Asparagus Officinalis L. roots and/or shoots, utmost preferred in an amount of from 0.5 to 5 I of methanol and/or ethanol and/or acetone per 100 g of the dry powder of Asparagus Officinalis L. roots and/or shoots.

6. The saponine-free extract of any of the claims 1 to 3, wherein in step b) there is used as the solvent a mixture of water and at least one organic liquid miscible with water and selected from the group consisting of straight-chain or branched or cyclic alcohols having 1 to 6 carbon atoms and straight-chain or branched or cyclic ketones having 3 to 6 carbon atoms, preferably a mixture of water and an organic liquid selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms, even more preferable a mixture of water and an organic liquid selected from methanol and ethanol and acetone and mixtures thereof, even more preferable a mixture of water and up to 50 vol.-% of methanol and/or ethanol and/or acetone, even more preferred a mixture of water and 20 to 40 vol.-% of methanol and/or ethanol and/or acetone, even more preferred a mixture of water and 25 to 35 vol.% of methanol and/or ethanol and/or acetone, most preferred said solvent in an amount of from 0.5 to 5 l of the solvent per 100 g of the dry powder of Asparagus Officinalis L. roots and/or shoots, utmost preferred said solvent in an amount of from 0.5 to 5 1 of methanol and/or ethanol and/or acetone per 100 g of the dry powder of Asparagus Officinalis L. roots and/or shoots.

7. The saponine-free extract according to any of the claims 1 to 6, wherein step c) is carried out by shaking the suspension for a time in the range of 10 to 48 h and/or shaking said suspension at a temperature in the range of 10 to 25 °C.

8. The saponine-free extract according to any of the claims 1 to 7, wherein step d) for separating of insoluble and soluble constituents of the suspension is carried out by at least one step selected from the group consisting of centrifugation, decantation, filtration and combinations of those steps.

9. The saponine-free extract according to any of the claims 1 to 8, wherein step e) for removing the solvent from the solution is carried out by at least one step selected from the group consisting of evaporating the solvent partly and crystallizing the solute, evaporating the solvent completely and recovering the dry solute, freeze drying the solution and combinations of those steps.

10. The saponine-free extract of any of the claims 1 to 9, wherein in step (i) there is used a solvent comprising a mixture of water and a high content of at least one organic liquid miscible with water, preferably a solvent comprising water and a high content of at least one organic liquid selected from the group consisting of straight-chain or branched or cyclic alcohols having 1 to 6 carbon atoms and straight-chain or branched or cyclic ketones having 3 to 6 carbon atoms, more preferably a solvent comprising water and a high content of an organic liquid selected from the group consisting of straight-chain or branched aliphatic alcohols having 1 to 3 carbon atoms and straight-chain or branched aliphatic ketones having 3 to 5 carbon atoms, even more preferably a solvent comprising water and a high content of methanol and/or ethanol and/or acetone, even more preferred a solvent comprising water and more than 50 vol.- % of methanol and/or ethanol and/or acetone, even more preferred a solvent comprising water and 60 to 90 vol.-% of methanol and/or ethanol and/or acetone, most preferred a solvent comprising water and 75 to 95 vol.-% of methanol and/or ethanol and/or acetone or by using, as a solvent, a purely organic liquid, preferably an organic liquid selected from the group consisting of straight-chain or branched or cyclic alcohols having 1 to 6 carbon atoms and straight-chain or branched or cyclic ketones having 3 to 6 carbon atoms, more preferably an organic liquid selected from the group consisting of straight-chain or branched aliphatic alcohols having 1 to 3 carbon atoms and straight-chain or branched aliphatic ketones having 3 to 5 carbon atoms, even more preferably an organic liquid selected from the group consisting of methanol and/or ethanol and/or acetone, most preferred ethanol.

11. The saponine-free extract of any of claims 1 to 10, wherein step (ii) is carried out by shaking the suspension or solution, preferably by shaking the suspension or solution for a time in the range of from 30 to 90 min and/or shaking the suspension or solution at a temperature in the range of from 10 to 25 °C.

12. The saponine-free extract of any of claims 1 to 11, wherein step (iii) for separating insoluble and soluble constituents of the suspension is carried out by at least one step selected from the group consisting of centrifugation, decantation, filtration and combinations of those steps.

13. The saponine-free extract of any of claims 1 to 12, wherein step (iv) is carried out by removing the solvent from the solution by at least one step selected from the group consisting of evaporating the solvent partly and crystallizing the solute, evaporating the solvent completely and recovering the dry solute, freeze drying the solution and combinations of those steps.

14. The saponine-free extract of any of claims 1 to 13, obtainable from an aqueous raw extract from Asparagus Officinalis L. roots.

15. The saponine-free extract of any of claims 1 to 13, obtainable from a hydro-alcoholic, preferably from a hydroethanolic, more preferably from a hydro-(20-to 40- vol.-% ethanolic) raw extract from Asparagus Officinalis L. roots.

16. The saponine-free extract of any of claims 1 to 13, obtainable from an aqueous raw extract from Asparagus Officinalis L. shoots.

17. The saponine-free extract of any of claims 1 to 13, obtainable from a hydro-alcoholic, preferably from a hydroethanolic, more preferably from a hydro-(20-to 40- vol.-% ethanolic) raw extract from Asparagus Officinalis L. shoots.

18. A process for preparing a saponine-free extract from roots and/or shoots of Asparagus Officinalis, comprising at least the steps of
a) grinding dry roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and
f) optionally grinding the raw extract obtained to prepare a raw extract of roots and/or shoots of Asparagus Officinalis L. in the form of a powder;
(i) adding at least one solvent selected from the group consisting of organic liquids miscible with water and mixtures of water and at least one organic liquid miscible with water, wherein the solvent has a lower hydrophilicity than the solvent used for the preparation of the raw extract, to the solution of soluble constituents of the raw extract obtained in step d) to obtain a solution or suspension;
(ii) agitating the solution or suspension so obtained for a time > 30 min and at a temperature not exposing the constituents of said suspension to the risk of a biological degradation;
(iii) separating the liquid phases in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases;
(iv) removing, from the liquid phase with the solvent of relatively high hydrophilicity, the solvent to obtain a solid residue; and
(v) drying and optionally grinding the solid residue to obtain a fractioned saponine-free extract from roots and/or shoots of Asparagus Officinalis L..

19. A process for preparing a saponine-free extract from roots and/or shoots of Asparagus Officinalis, comprising at least the steps of
a) grinding dry roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and
f) optionally grinding the raw extract obtained to prepare a raw extract of roots and/or shoots of Asparagus Officinalis L. in the form of a powder;
(i) adding at least one solvent selected from the group consisting of organic liquids miscible with water and mixtures of water and at least one organic liquid miscible with water, wherein the solvent has a lower hydrophilicity than the solvent used for the preparation of the raw extract, to a solution of the raw extract obtained in step e) and/or step f) in a solvent selected from the group of water and the solvent which was used for obtaining the raw extract in steps a) to d), to obtain a solution or suspension;
(ii) agitating the solution or suspension so obtained for a time > 30 min and at a temperature not exposing the constituents of said suspension to the risk of a biological degradation;
(iii) separating the liquid phases in accordance with a suitable parameter thereof from each other and, optionally, from any insoluble constituent to obtain a liquid phase with a solvent of relatively high hydrophilicity and a liquid phase with a solvent of relatively lower hydrophilicity and, optionally a solid phase insoluble in both liquid phases;
(iv) removing, from the liquid phase with the solvent of relatively high hydrophilicity, the solvent to obtain a solid residue; and
(v) drying and optionally grinding the solid residue to obtain a fractioned saponine-free extract from roots and/or shoots of Asparagus Officinalis L..

20. A process for preparing a saponine-free extract from roots and/or shoots of Asparagus Officinalis, comprising at least the steps of
a) grinding dry roots and/or shoots of Asparagus Officinalis L. to obtain a dry powder;
b) adding to the dry powder so obtained a solvent selected from the group consisting of water, at least one organic liquid miscible with water and mixtures of water and at least one organic liquid miscible with water to obtain a suspension;
c) agitating the suspension so obtained for a time > 10 h and at a temperature not exposing the constituents of said suspension to the risk of a biological degradation;
d) separating the constituents of the suspension not soluble in said solvent from those constituents soluble in said solvent to obtain a soluble fraction dissolved in the solvent and an insoluble fraction; and
e) removing the solvent from the soluble fraction to obtain a raw extract; and
f) optionally grinding the raw extract obtained to prepare a raw extract of roots and/or shoots of Asparagus Officinalis L. in the form of a powder;
(i) adding at least one solvent selected from the group consisting of organic liquids miscible with water and mixtures of water and at least one organic liquid miscible with water, wherein the solvent has a lower hydrophilicity than the solvent used for the preparation of the raw extract, to the raw extract obtained in step e) and/or f) to obtain a suspension;
(ii) agitating the suspension so obtained for a time > 30 min and at a temperature not exposing the constituents of said suspension to the risk of a biological degradation;
(iii) separating the liquid phase from any insoluble constituent to obtain a solid residue insoluble in the liquid phase; and
(iv) drying and optionally grinding the solid residue to obtain a fractioned saponine-free extract from roots and/or shoots of Asparagus Officinalis L..

21. The process of any of claims 18 to 20, wherein in step b) water is used as the solvent, preferably wherein water is used as the solvent in an amount of from 0.1 to 10 l of water per 100 g of a dry powder of Asparagus Officinalis L. roots and/or shoots, more preferred wherein water is used as the solvent in an amount of from 0.5 to 5 l of water per 100 g of a dry powder of Asparagus Officinalis L. roots and/or shoots.

22. The process of any of claims 18 to 20, wherein in step b) there is used as the solvent at least one organic liquid miscible with water and selected from the group consisting of straight-chain or branched or cyclic alcohols having 1 to 6 carbon atoms and straight-chain or branched or cyclic ketones having 3 to 6 carbon atoms is used as the solvent, preferably wherein at least one organic liquid selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms is used as the solvent, even more preferable wherein at least one organic liquid selected from methanol and ethanol and acetone and mixtures thereof is used as the solvent, most preferred wherein at least one of said solvents is used in an amount of from 0.5 to 5 l of the solvent per 100 g of the dry powder of Asparagus Officinalis L. roots and/or shoots, utmost preferred in an amount of from 0.5 to 5 l of methanol and/or ethanol and/or acetone per 100 g of the dry powder of Asparagus Officinalis L. roots and/or shoots.

23. The process of any of claims 18 to 20, wherein in step b) there is used as the solvent a mixture of water and at least one organic liquid miscible with water and selected from the group consisting of straight-chain or branched or cyclic alcohols having 1 to 6 carbon atoms and straight-chain or branched or cyclic ketones having 3 to 6 carbon atoms is used as the solvent, preferably wherein a mixture of water and an organic liquid selected from the group consisting of aliphatic straight-chain or branched alcohols having 1 to 6 carbon atoms, more preferably having 1 to 3 carbon atoms, and straight-chain or branched aliphatic ketones having 3 to 5 carbon atoms, is used as the solvent, even more preferable wherein a mixture of water and an organic liquid selected from methanol and ethanol and acetone and mixtures thereof is used as the solvent, even more preferable wherein a mixture of water and up to 50 vol.-% of methanol and/or ethanol and/or acetone is used as the solvent, even more preferred wherein a mixture of water and 20 to 40 vol.-% of methanol and/or ethanol and/or acetone, even more preferred a mixture of water and 25 to 35 vol.-% of methanol and/or ethanol and/or acetone, is used as the solvent; most preferred wherein said solvent is used in an amount of from 0.5 to 5 l of the solvent per 100 g of the dry powder of Asparagus Officinalis L. roots and/or shoots, utmost preferred said solvent is used in an amount of from 0.5 to 5 l of methanol and/or ethanol and/or acetone per 100 g of the dry powder of Asparagus Officinalis L. roots and/or shoots.

24. The process of any of claims 18 to 23, wherein in step c) the agitating of the suspension is a shaking of the suspension, preferably a shaking of the suspension for a time in the range of from 10 to 48 h and/or a shaking of the suspension at a temperature in the range of from 10 to 25 °C.

25. The process of any of claims 18 to 24, wherein in step d) insoluble and soluble constituents of the suspension are separated by at least one step selected from the group consisting of centrifugation, decantation, filtration and combinations of those steps.

26. The process of any of claims 18 to 25, wherein in step e) the solvent is removed from the solution by at least one step selected from the group consisting of evaporating the solvent partly and crystallizing the solute, evaporating the solvent completely and recovering the dry solute, freeze drying the solution and combinations of those steps.

27. The process of any of claims 18 to 26, wherein in step (i) a solvent is used comprising a mixture of water and a high content of at least one organic liquid miscible with water, preferably wherein a solvent comprising water and a high content of at least one organic liquid selected from the group consisting of straight-chain or branched or cyclic alcohols having 1 to 6 carbon atoms and straight-chain or branched or cyclic ketones having 3 to 6 carbon atoms is used, more preferably wherein a solvent comprising water and a high content of an organic liquid selected from the group consisting of straight-chain or branched aliphatic alcohols having 1 to 3 carbon atoms and straight-chain or branched aliphatic ketones having 3 to 5 carbon atoms is used, even more preferably wherein a solvent comprising water and a high content of methanol and/or ethanol and/or acetone is used, even more preferred wherein a solvent comprising water and more than 50 vol.-% of methanol and/or ethanol and/or acetone is used, even more preferred wherein a solvent comprising water and 60 to 90 vol.-% of methanol and/or ethanol and/or acetone, most preferred a solvent comprising water and 75 to 95 vol.-% of methanol and/or ethanol and/or acetone is used, or wherein, as a solvent, a purely organic liquid is used, preferably wherein an organic liquid selected from the group consisting of straight-chain or branched or cyclic alcohols having 1 to 6 carbon atoms and straight-chain or branched or cyclic ketones having 3 to 6 carbon atoms is used, more preferably wherein an organic liquid selected from the group consisting of straight-chain or branched aliphatic alcohols having 1 to 3 carbon atoms and straight-chain or branched aliphatic ketones having 3 to 5 carbon atoms is used, even more preferably wherein an organic liquid selected from the group consisting of methanol and/or ethanol and/or acetone, most preferred ethanol, is used.

28. The process of any of the claims 18 to 27, wherein in step (ii) the agitating the suspension or solution is a shaking the suspension or solution, preferably by shaking the suspension or solution for a time in the range of from 30 to 90 min and/or shaking the suspension or solution at a temperature in the range of from 10 to 25 °C.

29. The process of any of the claims 18 to 28, wherein in step (iii) insoluble and soluble constituents of the suspension are separated by at least one step selected from the group consisting of centrifugation, decantation, filtration and combinations of those steps.

30. The process of any of the claims 18 to 29, wherein in step (iv) the solvent is removed from the solution by at least one step selected from the group consisting of evaporating the solvent partly and crystallizing the solute, evaporating the solvent completely and recovering the dry solute, freeze drying the solution and combinations of those steps.

31. The process of any of claims 18 to 30, wherein Asparagus Officinalis L. roots are used as the starting material.

32. The process of any of claims 18 to 30, wherein an aqueous raw extract obtainable by the steps a) to e) from Asparagus Officinalis L. roots is used.

33. The process of any of claims 18 to 30, wherein a hydro-alcoholic, preferably a hydroethanolic, more preferably a hydro-(20- to 40- vol.-% ethanolic) raw extract obtainable by the steps a) to e) from Asparagus Officinalis L. roots is used.

34. The process of any of claims 18 to 30, wherein Asparagus Officinalis L. shoots are used as the starting material.

35. The process of any of claims 18 to 30, wherein an aqueous raw extract obtainnable by the steps a) to e) from Asparagus Officinalis L. shoots is used.

36. The process of any of the claims 18 to 30, wherein a hydro-alcoholic, preferably a hydroethanolic, more preferably a hydro-(20- to 40- vol.-% ethanolic) raw extract obtainable by the steps a) to e) from Asparagus Officinalis L. shoots is used.

37. Pharmaceutical preparation or nutritional supplement, in particular vegetable diuretic preparation, comprising one or more of saponine-free extracts from Asparagus Officinalis L. roots and/or shoots according to any of claims 1 to 17, optionally together with one or more pharmacologically acceptable carrier substances, auxiliary substances and/or vehicles.

38. Pharmaceutical preparation or nutritional supplement, in particular vegetable diuretic preparation, according to claim 37, comprising the saponine-free ethanol-insoluble component of the aqueous or of the hydro-alcoholic raw extract of Asparagus Officinalis L. roots and/or shoots.

39. The saponine-free extract from roots and/or shoots of Asparagus Officinalis L. according to any of claims 1 to 17 for medical use.

40. The use of one or more of saponine-free extracts from Asparagus Officinalis L. roots and/or shoots according to any of claims 1 to 17, for the manufacture of a medicament for the promotion of a diuresis, for the promotion of purification of the body, for the promotion of body weight reduction, for the prevention and/or treatment of heart failure, for the prevention and/or treatment of hypertension and/or for the prevention and/or treatment of heart hypertrophia.

## Patentansprüche

1. Saponin-freier Extrakt aus Wurzeln und/oder Sprossen von Asparagus Officinalis L., erhältlich durch wenigstens die Schritte:
(a) Mahlen trockener Wurzeln und/oder Sprossen von Asparagus Officinalis L. unter Erhalt eines trockenen Pulvers;
(b) Zugabe eines Lösungsmittels zu dem so erhaltenen trockenen Pulver, das ausgewählt ist aus der Gruppe, die besteht aus Wasser, wenigstens einer organischen Flüssigkeit, die mit Wasser mischbar ist, und Mischungen aus Wasser und wenigstens einer organischen Flüssigkeit, die mit Wasser mischbar ist, unter Erhalt einer Suspension;
(c) Rühren der so erhaltenen Suspension für eine Zeit von > 10 h und bei einer Temperatur, bei der die Bestandteile der Suspension nicht dem Risiko eines biologischen Abbaus ausgesetzt werden;
(d) Trennen der Bestandteile der Suspension, die in dem Lösungsmittel nicht löslich sind, von denjenigen Bestandteilen der Suspension, die in dem Lösungsmittel löslich sind, unter Erhalt einer löslichen Fraktion, die in dem Lösungsmittel gelöst ist und einer unlöslichen Fraktion; und
(e) Entfernen des Lösungsmittels von der löslichen Fraktion unter Erhalt eines Rohextrakts; und
(f) gegebenenfalls Mahlen des erhaltenen Rohextrakts zur Herstellung eines Rohextrakts von Wurzeln und/oder Sprossen von Asparagus Officinalis L. in Form eines Pulvers;
(i) Zugabe von wenigstens einem Lösungsmittel, das ausgewählt ist aus der Gruppe, die besteht aus organischen Flüssigkeiten, die mit Wasser mischbar sind, und Mischungen aus Wasser und wenigstens einer organischen Flüssigkeit, die mit Wasser mischbar ist, worin das Lösungsmittel eine geringere Hydrophilie aufweist als das Lösungsmittel, das verwendet wurde für die Herstellung des Rohextrakts, zu der Lösung löslicher Bestandteile des Rohextrakts, der in Schritt (d) erhalten wurde, unter Erhalt einer Lösung oder Suspension;
(ii) Rühren der so erhaltenen Lösung oder Suspension für eine Zeit von > 30 min und bei einer Temperatur, bei der die Bestandteile der Suspension nicht dem Risiko eines biologischen Abbaus ausgesetzt werden;
(iii) Trennen der flüssigen Phasen voneinander in Übereinstimmung mit einem geeigneten Parameter davon und gegebenenfalls von irgendeinem unlöslichen Bestandteil unter Erhalt einer flüssigen Phase mit einem Lösungsmittel relativ hoher Hydrophilie und einer flüssigen Phase mit einem Lösungsmittel relativ niedriger Hydrophilie und gegebenenfalls einer festen Phase, die in beiden flüssigen Phasen unlöslich ist;
(iv) Entfernen des Lösungsmittels von der flüssigen Phase mit dem Lösungsmittel relativ hoher Hydrophilie unter Erhalt eines festen Rückstands; und
(v) Trocknen und gegebenenfalls Mahlen des festen Rückstands unter Erhalt eines fraktionierten Saponin-freien Extrakts aus Wurzeln und/oder Sprossen von Asparagus Officinalis L..

2. Saponin-freier Extrakt aus Wurzeln und/oder Sprossen von Asparagus Officinalis L., erhältlich durch wenigstens die Schritte:
(a) Mahlen trockener Wurzeln und/oder Sprossen von Asparagus Officinalis L. unter Erhalt eines trockenen Pulvers;
(b) Zugabe eines Lösungsmittels zu dem so erhaltenen trockenen Pulver, das ausgewählt ist aus der Gruppe, die besteht aus Wasser, wenigstens einer organischen Flüssigkeit, die mit Wasser mischbar ist, und Mischungen aus Wasser und wenigstens einer organischen Flüssigkeit, die mit Wasser mischbar ist, unter Erhalt einer Suspension;
(c) Rühren der so erhaltenen Suspension für eine Zeit von > 10 h und bei einer Temperatur, bei der die Bestandteile der Suspension nicht dem Risiko eines biologischen Abbaus ausgesetzt werden;
(d) Trennen der Bestandteile der Suspension, die in dem Lösungsmittel nicht löslich sind, von denjenigen Bestandteilen der Suspension, die in dem Lösungsmittel löslich sind, unter Erhalt einer löslichen Fraktion, die in dem Lösungsmittel gelöst ist und einer unlöslichen Fraktion; und
(e) Entfernen des Lösungsmittels von der löslichen Fraktion unter Erhalt eines Rohextrakts; und
(f) gegebenenfalls Mahlen des erhaltenen Rohextrakts zur Herstellung eines Rohextrakts von Wurzeln und/oder Sprossen von Asparagus Officinalis L. in Form eines Pulvers;
(i) Zugabe von wenigstens einem Lösungsmittel, das ausgewählt ist aus der Gruppe, die besteht aus organischen Flüssigkeiten, die mit Wasser mischbar sind, und Mischungen aus Wasser und wenigstens einer organischen Flüssigkeit, die mit Wasser mischbar ist, worin das Lösungsmittel eine geringere Hydrophilie aufweist als das Lösungsmittel, das verwendet wurde für die Herstellung des Rohextrakts, zu einer Lösung des Rohextrakts, der in Schritt (e) und/oder Schritt (f) erhalten wurde, in einem Lösungsmittel, das ausgewählt ist aus der Gruppe Wasser und das Lösungsmittel, das in den Schritten (a) bis (d) verwendet wurde, um den Rohextrakt zu erhalten, unter Erhalt einer Lösung oder Suspension;
(ii) Rühren der so erhaltenen Lösung oder Suspension für eine Zeit von > 30 min und bei einer Temperatur, bei der die Bestandteile der Suspension nicht dem Risiko eines biologischen Abbaus ausgesetzt werden;
(iii) Trennen der flüssigen Phasen voneinander in Übereinstimmung mit einem geeigneten Parameter davon und gegebenenfalls von irgendeinem unlöslichen Bestandteil unter Erhalt einer flüssigen Phase mit einem Lösungsmittel relativ hoher Hydrophilie und einer flüssigen Phase mit einem Lösungsmittel relativ niedriger Hydrophilie und gegebenenfalls einer festen Phase, die in beiden flüssigen Phasen unlöslich ist;
(iv) Entfernen des Lösungsmittels von der flüssigen Phase mit dem Lösungsmittel relativ hoher Hydrophilie unter Erhalt eines festen Rückstands; und
(v) Trocknen und gegebenenfalls Mahlen des festen Rückstands unter Erhalt eines fraktionierten Saponin-freien Extrakts aus Wurzeln und/oder Sprossen von Asparagus Officinalis L..

3. Saponin-freier Extrakt aus Wurzeln und/oder Sprossen von Asparagus Officinalis L., erhältlich durch wenigstens die Schritte:
(a) Mahlen trockener Wurzeln und/oder Sprossen von Asparagus Officinalis L. unter Erhalt eines trockenen Pulvers;
(b) Zugabe eines Lösungsmittels zu dem so erhaltenen trockenen Pulver, das ausgewählt ist aus der Gruppe, die besteht aus Wasser, wenigstens einer organischen Flüssigkeit, die mit Wasser mischbar ist, und Mischungen aus Wasser und wenigstens einer organischen Flüssigkeit, die mit Wasser mischbar ist, unter Erhalt einer Suspension;
(c) Rühren der so erhaltenen Suspension für eine Zeit von > 10 h und bei einer Temperatur, bei der die Bestandteile der Suspension nicht dem Risiko eines biologischen Abbaus ausgesetzt werden;
(d) Trennen der Bestandteile der Suspension, die in dem Lösungsmittel nicht löslich sind, von denjenigen Bestandteilen der Suspension, die in dem Lösungsmittel löslich sind, unter Erhalt einer löslichen Fraktion, die in dem Lösungsmittel gelöst ist und einer unlöslichen Fraktion; und
(e) Entfernen des Lösungsmittels von der löslichen Fraktion unter Erhalt eines Rohextrakts; und
(f) gegebenenfalls Mahlen des erhaltenen Rohextrakts zur Herstellung eines Rohextrakts von Wurzeln und/oder Sprossen von Asparagus Officinalis L. in Form eines Pulvers;
(i) Zugabe von wenigstens einem Lösungsmittel, das ausgewählt ist aus der Gruppe, die besteht aus organischen Flüssigkeiten, die mit Wasser mischbar sind und Mischungen aus Wasser und wenigstens einer organischen Flüssigkeit, die mit Wasser mischbar ist, worin das Lösungsmittel eine geringere Hydrophilie aufweist als das Lösungsmittel, das verwendet wurde für die Herstellung des Rohextrakts, zu dem Rohextrakt, der in Schritt (e) und/oder Schritt (f) erhalten wurde, unter Erhalt einer Suspension;
(ii) Rühren der so erhaltenen Suspension für eine Zeit von > 30 min und bei einer Temperatur, bei der die Bestandteile der Suspension nicht dem Risiko eines biologischen Abbaus ausgesetzt werden;
(iii) Trennen der flüssigen Phase von irgendeinem unlöslichen Bestandteil unter Erhalt eines festen Rückstands, der in der flüssigen Phase unlöslich ist; und
(iv) Trocknen und gegebenenfalls Mahlen des festen Rückstands unter Erhalt eines fraktionierten Saponin-freien Extrakts aus Wurzeln und/oder Sprossen von Asparagus Officinalis L..

4. Saponin-freier Extrakt nach einem der Ansprüche 1 bis 3, worin in Schritt (b) Wasser als Lösungsmittel verwendet wird, vorzugsweise Wasser als Lösungsmittel in einer Menge von 0,1 bis 101 Wasser pro 100 g eines trockenen Pulvers der Asparagus Officinalis L.-Wurzeln und/oder -Sprossen, noch mehr bevorzugt Wasser als Lösungsmittel in einer Menge von 0,5 bis 51 Wasser pro 100 g eines trockenen Pulvers der Asparagus Officinalis L.-Wurzeln und/oder -Sprossen.

5. Saponin-freier Extrakt nach einem der Ansprüche 1 bis 3, worin in Schritt (b) als Lösungsmittel wenigstens eine organische Flüssigkeit verwendet wird, die mit Wasser mischbar ist, und gewählt ist aus der Gruppe, die besteht aus geradkettigen oder verzweigten oder cyclischen Alkoholen mit 1 bis 6 Kohlenstoffatomen und geradkettigen oder verzweigten oder cyclischen Ketonen mit 3 bis 6 Kohlenstoffatomen, vorzugsweise ausgewählt aus der Gruppe, die besteht aus aliphatischen geradkettigen oder verzweigten Alkoholen mit 1 bis 6 Kohlenstoffatomen, noch mehr bevorzugt 1 bis 3 Kohlenstoffatomen, und geradkettigen oder verzweigten aliphatischen Ketonen mit 3 bis 5 Kohlenstoffatomen, noch mehr bevorzugt ausgewählt aus Methanol und Ethanol und Aceton und Mischungen daraus, am meisten bevorzugt in einer Menge von 0,5 bis 5 1 Lösungsmittel pro 100 g des trockenen Pulvers von Asparagus Officinalis L.-Wurzeln und/oder -Sprossen, am allermeisten bevorzugt in einer Menge von 0,5 bis 51 Methanol und/oder Ethanol und/oder Aceton pro 100 g des trockenen Pulvers von Asparagus Officinalis-L.-Wurzeln und/oder -Sprossen.

6. Saponin-freier Extrakt nach einem der Ansprüche 1 bis 3, worin in Schritt (b) als Lösungsmittel eine Mischung aus Wasser und wenigsten einer organischen Flüssigkeit verwendet wird, die mit Wasser mischbar ist, und ausgewählt ist aus der Gruppe, die besteht aus geradkettigen oder verzweigten oder cyclischen Alkoholen mit 1 bis 6 Kohlenstoffatomen und geradkettigen oder verzweigten oder cyclischen Ketonen mit 3 bis 6 Kohlenstoffatomen, vorzugsweise eine Mischung aus Wasser und einer organischen Flüssigkeit, die ausgewählt ist aus der Gruppe, die besteht aus aliphatischen geradkettigen oder verzweigten Alkoholen mit 1 bis 6 Kohlenstoffatomen, noch mehr bevorzugt 1 bis 3 Kohlenstoffatomen, und geradkettigen oder verzweigten aliphatischen Ketonen mit 3 bis 5 Kohlenstoffatomen, noch mehr bevorzugt eine Mischung aus Wasser und einer organischen Flüssigkeit, ausgewählt aus Methanol und Ethanol und Aceton und Mischungen daraus, noch mehr bevorzugt eine Mischung aus Wasser und bis zu 50 Vol.-% Methanol und/oder Ethanol und/oder Aceton, noch mehr bevorzugt eine Mischung aus Wasser und 20 bis 40 Vol-% Methanol und/oder Ethanol und/oder Aceton, noch mehr bevorzugt eine Mischung aus Wasser und 25 bis 35 Vol-% Methanol und/oder Ethanol und/oder Aceton, am meisten bevorzugt das Lösungsmittel in einer Menge von 0,5 bis 5 l Lösungsmittel pro 100 g des trockenen Pulvers von Asparagus Officinalis L.-Wurzeln und/oder -Sprossen, am allermeisten bevorzugt das Lösungmittel in einer Menge von 0,5 bis 5 l Methanol und/oder Ethanol und/oder Aceton pro 100 g des trockenen Pulvers von Asparagus Officinalis-L.-Wurzeln und/oder -Sprossen verwendet wird.

7. Saponin-freier Extrakt nach einem der Ansprüche 1 bis 6, worin Schritt (c) durchgeführt wird durch Schütteln der Suspension für eine Zeit im Bereich von 10 bis 48 h und/oder Schütteln der Suspension bei einer Temperatur im Bereich von 10 bis 25 °C.

8. Saponin-freier Extrakt nach einem der Ansprüche 1 bis 7, worin Schritt (d) zum Trennen der unlöslichen und löslichen Bestandteile der Suspension durchgeführt wird durch wenigstens einen Schritt, der ausgewählt ist aus der Gruppe, die besteht aus Zentrifugieren, Dekantieren, Filtrieren und Kombinationen dieser Schritte.

9. Saponin-freier Extrakt nach einem der Ansprüche 1 bis 8, worin Schritt (e) zum Entfernen des Lösungsmittels von der Lösung durchgeführt wird durch wenigstens einen Schritt, der ausgewählt ist aus der Gruppe, die besteht aus teilweisem Verdampfen des Lösungsmittels und Kristallisieren der gelösten Substanz, vollständigem Verdampfen des Lösungsmittels und Gewinnen der trockenen gelösten Substanz, Gefriertrocknen der Lösung und Kombinationen dieser Schritte.

10. Saponin-freier Extrakt nach einem der Ansprüche 1 bis 9, worin in Schritt (i) ein Lösungsmittel verwendet wird, das eine Mischung aus Wasser und einem hohen Gehalt an wenigsten einer organischen Flüssigkeit, die mit Wasser mischbar ist, umfasst, vorzugsweise ein Lösungsmittel, das Wasser und einen hohen Gehalt an wenigstens einer organischen Flüssigkeit umfasst, die ausgewählt ist aus der Gruppe, die besteht aus geradkettigen oder verzweigten oder cyclischen Alkoholen mit 1 bis 6 Kohlenstoffatomen und geradkettigen oder verzweigten oder cyclischen Ketonen mit 3 bis 6 Kohlenstoffatomen, noch mehr bevorzugt ein Lösungsmittel, das Wasser und einen hohen Gehalt an einer organischen Flüssigkeit umfasst, die ausgewählt ist aus der Gruppe, die besteht aus geradkettigen oder verzweigten aliphatischen Alkoholen mit 1 bis 3 Kohlenstoffatomen und geradkettigen oder verzweigten aliphatischen Ketonen mit 3 bis 5 Kohlenstoffatomen, noch mehr bevorzugt ein Lösungsmittel, das Wasser und einen hohen Gehalt an Methanol und/oder Ethanol und/oder Aceton umfasst, noch mehr bevorzugt ein Lösungsmittel, das Wasser und mehr als 50 Vol-% Methanol und/oder Ethanol und/oder Aceton umfasst, noch mehr bevorzugt ein Lösungsmittel, das Wasser und 60 bis 90 Vol-% Methanol und/oder Ethanol und/oder Aceton umfasst, am meisten bevorzugt ein Lösungsmittel, das Wasser und 75 bis 95 Vol-% Methanol und/oder Ethanol und/oder Aceton umfasst, oder als Lösungsmittel eine rein organische Flüssigkeit verwendet wird, vorzugsweise eine organische Flüssigkeit, die ausgewählt ist aus der Gruppe, die besteht aus geradkettigen oder verzweigen oder cyclischen Alkoholen mit 1 bis 6 Kohlenstoffatomen und geradkettigen oder verzweigten oder cyclischen Ketonen mit 3 bis 6 Kohlenstoffatomen, noch mehr bevorzugt eine organische Flüssigkeit, die ausgewählt ist aus der Gruppe, die besteht aus geradkettigen oder verzweigten aliphatischen Alkoholen mit 1 bis 3 Kohlenstoffatomen und geradkettigen oder verzweigten aliphatischen Ketonen mit 3 bis 5 Kohlenstoffatomen, noch mehr bevorzugt eine organische Flüssigkeit, die ausgewählt ist aus der Gruppe, die besteht aus Methanol und/oder Ethanol und/oder Aceton, am meisten bevorzugte Ethanol.

11. Saponin-freier Extrakt nach einem der Ansprüche 1 bis 10, worin Schritt (ii) durchgeführt wird durch Schütteln der Suspension oder Lösung, vorzugsweise durch Schütteln der Suspension oder Lösung für eine Zeit im Bereich von 30 bis 90 min und/oder Schütteln der Suspension oder Lösung bei einer Temperatur im Bereich von 10 bis 25 °C

12. Saponin-freier Extrakt nach einem der Ansprüche 1 bis 11, worin Schritt (iii) zum Trennen der unlöslichen und löslichen Bestandteile der Suspension durchgeführt wird durch wenigstens einen Schritt, der ausgewählt ist aus der Gruppe, die besteht aus Zentrifugieren, Dekantieren, Filtrieren und Kombinationen dieser Schritte.

13. Saponin-freier Extrakt nach einem der Ansprüche 1 bis 12, worin Schritt (iv) durchgeführt wird durch Entfernen des Lösungsmittels von der Lösung durchgeführt wird durch wenigstens einen Schritt, der ausgewählt ist aus der Gruppe, die besteht aus teilweisem Verdampfen des Lösungsmittels und Kristallisieren der gelösten Substanz, vollständigem Verdampfen des Lösungsmittels und Gewinnen der trockenen gelösten Substanz, Gefriertrocknen der Lösung und Kombinationen dieser Schritte.

14. Saponin-freier Extrakt nach einem der Ansprüche 1 bis 13, erhältlich aus einem wässrigen Rohextrakt aus Asparagus Officinalis L.-Wurzeln.

15. Saponin-freier Extrakt nach einem der Ansprüche 1 bis 13, erhältlich aus einem hydro-alkoholischen, vorzugsweise aus einem hydro-ethanolischen, noch mehr bevorzugt aus einem hydro-(20 bis 40 Vol-%-ethanolischen) Rohextrakt aus Asparagus Officinalis-L.-Wurzeln.

16. Saponin-freier Extrakt nach einem der Ansprüche 1 bis 13, erhältlich aus einem wässrigen Rohextrakt aus Asparagus Officinalis L.-Sprossen.

17. Saponin-freier Extrakt nach einem der Ansprüche 1 bis 13, erhältlich aus einem hydro-alkoholischen, vorzugsweise aus einem hydro-ethanolischen, noch mehr bevorzugt aus einem hydro-(20 bis 40 Vol-%-ethanolischen) Rohextrakt aus Asparagus Officinalis-L.-Sprossen.

18. Verfahren zur Herstellung eines Saponin-freien Extrakts aus Wurzeln und/oder Sprossen von Asparagus Officinalis L., das wenigstens die Schritte umfasst:
(a) Mahlen trockener Wurzeln und/oder Sprossen von Asparagus Officinalis L. unter Erhalt eines trockenen Pulvers;
(b) Zugabe eines Lösungsmittels zu dem so erhaltenen trockenen Pulver, das ausgewählt ist aus der Gruppe, die besteht aus Wasser, wenigstens einer organischen Flüssigkeit, die mit Wasser mischbar ist, und Mischungen aus Wasser und wenigstens einer organischen Flüssigkeit, die mit Wasser mischbar ist, unter Erhalt einer Suspension;
(c) Rühren der so erhaltenen Suspension für eine Zeit von > 10 h und bei einer Temperatur, bei der die Bestandteile der Suspension nicht dem Risiko eines biologischen Abbaus ausgesetzt werden;
(d) Trennen der Bestandteile der Suspension, die in dem Lösungsmittel nicht löslich sind, von denjenigen Bestandteilen der Suspension, die in dem Lösungsmittel löslich sind, unter Erhalt einer löslichen Fraktion, die in dem Lösungsmittel gelöst ist und einer unlöslichen Fraktion; und
(e) Entfernen des Lösungsmittels von der löslichen Fraktion unter Erhalt eines Rohextrakts; und
(f) gegebenenfalls Mahlen des erhaltenen Rohextrakts zur Herstellung eines Rohextrakts von Wurzeln und/oder Sprossen von Asparagus Officinalis L. in Form eines Pulvers;
(i) Zugabe von wenigstens einem Lösungsmittel, das ausgewählt ist aus der Gruppe, die besteht aus organischen Flüssigkeiten, die mit Wasser mischbar sind, und Mischungen aus Wasser und wenigstens einer organischen Flüssigkeit, die mit Wasser mischbar ist, worin das Lösungsmittel eine geringere Hydrophilie aufweist als das Lösungsmittel, das verwendet wurde für die Herstellung des Rohextrakts, zu der Lösung löslicher Bestandteile des Rohextrakts, der in Schritt (d) erhalten wurde, unter Erhalt einer Lösung oder Suspension;
(ii) Rühren der so erhaltenen Lösung oder Suspension für eine Zeit von > 30 min und bei einer Temperatur, bei der die Bestandteile der Suspension nicht dem Risiko eines biologischen Abbaus ausgesetzt werden;
(iii) Trennen der flüssigen Phasen voneinander in Übereinstimmung mit einem geeigneten Parameter und gegebenenfalls von irgendeinem unlöslichen Bestandteil unter Erhalt einer flüssigen Phase mit einem Lösungsmittel relativ hoher Hydrophilie und einer flüssigen Phase mit einem Lösungsmittel relativ niedriger Hydrophilie und gegebenenfalls einer festen Phase, die in beiden flüssigen Phasen unlöslich ist;
(iv) Entfernen des Lösungsmittels von der flüssigen Phase mit dem Lösungsmittel relativ hoher Hydrophilie unter Erhalt eines festen Rückstands; und
(v) Trocknen und gegebenenfalls Mahlen des festen Rückstands unter Erhalt eines fraktionierten Saponin-freien Extrakts aus Wurzeln und/oder Sprossen von Asparagus Officinalis L..

19. Verfahren zur Herstellung eines Saponin-freien Extrakts aus Wurzeln und/oder Sprossen von Asparagus Officinalis L., das wenigstens die Schritte umfasst:
(a) Mahlen trockener Wurzeln und/oder Sprossen von Asparagus Officinalis L. unter Erhalt eines trockenen Pulvers;
(b) Zugabe eines Lösungsmittels zu dem so erhaltenen trockenen Pulver, das ausgewählt ist aus der Gruppe, die besteht aus Wasser, wenigstens einer organischen Flüssigkeit, die mit Wasser mischbar ist, und Mischungen aus Wasser und wenigstens einer organischen Flüssigkeit, die mit Wasser mischbar ist, unter Erhalt einer Suspension;
(c) Rühren der so erhaltenen Suspension für eine Zeit von > 10 h und bei einer Temperatur, bei der die Bestandteile der Suspension nicht dem Risiko eines biologischen Abbaus ausgesetzt werden;
(d) Trennen der Bestandteile der Suspension, die in dem Lösungsmittel nicht löslich sind, von denjenigen Bestandteilen der Suspension, die in dem Lösungsmittel löslich sind, unter Erhalt einer löslichen Fraktion, die in dem Lösungsmittel gelöst ist und einer unlöslichen Fraktion; und
(e) Entfernen des Lösungsmittels von der löslichen Fraktion unter Erhalt eines Rohextrakts; und
(f) gegebenenfalls Mahlen des erhaltenen Rohextrakts zur Herstellung eines Rohextrakts von Wurzeln und/oder Sprossen von Asparagus Officinalis L. in Form eines Pulvers;
(i) Zugabe von wenigstens einem Lösungsmittel, das ausgewählt ist aus der Gruppe, die besteht aus organischen Flüssigkeiten, die mit Wasser mischbar sind, und Mischungen aus Wasser und wenigstens einer organischen Flüssigkeit, die mit Wasser mischbar ist, worin das Lösungsmittel eine geringere Hydrophilie aufweist als das Lösungsmittel, das verwendet wurde für die Herstellung des Rohextrakts, zu einer Lösung des Rohextrakts, der in Schritt (e) und/oder Schritt (f) erhalten wurde, in einem Lösungsmittel, das ausgewählt ist aus der Gruppe Wasser und das Lösungsmittel, das in den Schritten (a) bis (d) verwendet wurde, um den Rohextrakt zu erhalten, unter Erhalt einer Lösung oder Suspension;
(ii) Rühren der so erhaltenen Lösung oder Suspension für eine Zeit von > 30 min und bei einer Temperatur, bei der die Bestandteile der Suspension nicht dem Risiko eines biologischen Abbaus ausgesetzt werden;
(iii) Trennen der flüssigen Phasen voneinander in Übereinstimmung mit einem geeigneten Parameter davon und gegebenenfalls von irgendeinem unlöslichen Bestandteil unter Erhalt einer flüssigen Phase mit einem Lösungsmittel relativ hoher Hydrophilie und einer flüssigen Phase mit einem Lösungsmittel relativ niedriger Hydrophilie und gegebenenfalls einer festen Phase, die in beiden flüssigen Phasen unlöslich ist;
(iv) Entfernen des Lösungsmittels von der flüssigen Phase mit dem Lösungsmittel relativ hoher Hydrophilie unter Erhalt eines festen Rückstands; und
(v) Trocknen und gegebenenfalls Mahlen des festen Rückstands unter Erhalt eines fraktionierten Saponin-freien Extrakts aus Wurzeln und/oder Sprossen von Asparagus Officinalis L..

20. Verfahren zur Herstellung eines Saponin-freien Extrakts aus Wurzeln und/oder Sprossen von Asparagus Officinalis L., das wenigstens die Schritte umfasst:
(a) Mahlen trockener Wurzeln und/oder Sprossen von Asparagus Officinalis L. unter Erhalt eines trockenen Pulvers;
(b) Zugabe eines Lösungsmittels zu dem so erhaltenen trockenen Pulver, das ausgewählt ist aus der Gruppe, die besteht aus Wasser, wenigstens einer organischen Flüssigkeit, die mit Wasser mischbar ist, und Mischungen aus Wasser und wenigstens einer organischen Flüssigkeit, die mit Wasser mischbar ist, unter Erhalt einer Suspension;
(c) Rühren der so erhaltenen Suspension für eine Zeit von > 10 h und bei einer Temperatur, bei der die Bestandteile der Suspension nicht dem Risiko eines biologischen Abbaus ausgesetzt werden;
(d) Trennen der Bestandteile der Suspension, die in dem Lösungsmittel nicht löslich sind, von denjenigen Bestandteilen der Suspension, die in dem Lösungsmittel löslich sind, unter Erhalt einer löslichen Fraktion, die in dem Lösungsmittel gelöst ist und einer unlöslichen Fraktion; und
(e) Entfernen des Lösungsmittels von der löslichen Fraktion unter Erhalt eines Rohextrakts; und
(f) gegebenenfalls Mahlen des erhaltenen Rohextrakts zur Herstellung eines Rohextrakts von Wurzeln und/oder Sprossen von Asparagus Officinalis L. in Form eines Pulvers;
(i) Zugabe von wenigstens einem Lösungsmittel, das ausgewählt ist aus der Gruppe, die besteht aus organischen Flüssigkeiten, die mit Wasser mischbar sind und Mischungen aus Wasser und wenigstens einer organischen Flüssigkeit, die mit Wasser mischbar ist, worin das Lösungsmittel eine geringere Hydrophilie aufweist als das Lösungsmittel, das verwendet wurde für die Herstellung des Rohextrakts, zu dem Rohextrakt, der in Schritt (e) und/oder Schritt (f) erhalten wurde, unter Erhalt einer Suspension;
(ii) Rühren der so erhaltenen Suspension für eine Zeit von > 30 min und bei einer Temperatur, bei der die Bestandteile der Suspension nicht dem Risiko eines biologischen Abbaus ausgesetzt werden;
(iii) Trennen der flüssigen Phase von irgendeinem unlöslichen Bestandteil unter Erhalt eines festen Rückstands, der in der flüssigen Phase unlöslich ist; und
(iv) Trocknen und gegebenenfalls Mahlen des festen Rückstands unter Erhalt eines fraktionierten Saponin-freien Extrakts aus Wurzeln und/oder Sprossen von Asparagus Officinalis L..

21. Verfahren nach einem der Ansprüche 18 bis 20, worin in Schritt (b) Wasser als Lösungsmittel verwendet wird, vorzugsweise worin Wasser als Lösungsmittel verwendet wird in einer Menge von 0,1 bis 101 Wasser pro 100 g eines trockenen Pulvers der Asparagus Officinalis L.-Wurzeln und/oder -Sprossen, noch mehr bevorzugt worin Wasser als Lösungsmittel verwendet wird in einer Menge von 0,5 bis 5 l Wasser pro 100 g eines trockenen Pulvers der Asparagus Officinalis L.-Wurzeln und/oder -Sprossen.

22. Verfahren nach einem der Ansprüche 18 bis 20, worin in Schritt (b) als Lösungsmittel wenigstens eine organische Flüssigkeit, die mit Wasser mischbar ist, und gewählt ist aus der Gruppe, die besteht aus geradkettigen oder verzweigten oder cyclischen Alkoholen mit 1 bis 6 Kohlenstoffatomen und geradkettigen oder verzweigten oder cyclischen Ketonen mit 3 bis 6 Kohlenstoffatomen als Lösungsmittel verwendet wird, vorzugsweise worin wenigstens eine organische Flüssigkeit, die ausgewählt ist aus der Gruppe, die besteht aus aliphatischen geradkettigen oder verzweigten Alkoholen mit 1 bis 6 Kohlenstoffatomen, noch mehr bevorzugt 1 bis 3 Kohlenstoffatomen, und geradkettigen oder verzweigten aliphatischen Ketonen mit 3 bis 5 Kohlenstoffatomen als Lösungsmittel verwendet wird, noch mehr bevorzugt worin wenigstens eine organische Flüssigkeit, ausgewählt aus Methanol und Ethanol und Aceton und Mischungen daraus als Lösungsmittel verwendet wird, am meisten bevorzugt worin wenigstens eines der genannten Lösungsmittel verwendet wird in einer Menge von 0,5 bis 5 l Lösungsmittel pro 100 g des trockenen Pulvers von Asparagus Officinalis L.-Wurzeln und/oder - Sprossen, am allermeisten bevorzugt in einer Menge von 0,5 bis 5 l Methanol und/oder Ethanol und/oder Aceton pro 100 g des trockenen Pulvers von Asparagus Officinalis-L.-Wurzeln und/oder -Sprossen.

23. Verfahren nach einem der Ansprüche 18 bis 20, worin in Schritt (b) als Lösungsmittel eine Mischung aus Wasser und wenigsten einer organischen Flüssigkeit, die mit Wasser mischbar ist, und ausgewählt ist aus der Gruppe, die besteht aus geradkettigen oder verzweigten oder cyclischen Alkoholen mit 1 bis 6 Kohlenstoffatomen und geradkettigen oder verzweigten oder cyclischen Ketonen mit 3 bis 6 Kohlenstoffatomen als Lösungsmittel verwendet wird, vorzugsweise worin eine Mischung aus Wasser und einer organischen Flüssigkeit, die ausgewählt ist aus der Gruppe, die besteht aus aliphatischen geradkettigen oder verzweigten Alkoholen mit 1 bis 6 Kohlenstoffatomen, noch mehr bevorzugt 1 bis 3 Kohlenstoffatomen, und geradkettigen oder verzweigten aliphatischen Ketonen mit 3 bis 5 Kohlenstoffatomen als Lösungsmittel verwendet wird, noch mehr bevorzugt worin eine Mischung aus Wasser und einer organischen Flüssigkeit, ausgewählt aus Methanol und Ethanol und Aceton und Mischungen daraus als Lösungsmittel verwendet wird, noch mehr bevorzugt worin eine Mischung aus Wasser und bis zu 50 Vol.-% Methanol und/oder Ethanol und/oder Aceton als Lösungsmittel verwendet wird, noch mehr bevorzugt worin eine Mischung aus Wasser und 20 bis 40 Vol-% Methanol und/oder Ethanol und/oder Aceton, noch mehr bevorzugt eine Mischung aus Wasser und 25 bis 35 Vol-% Methanol und/oder Ethanol und/oder Aceton als Lösungsmittel verwendet wird, am meisten bevorzugt worin das Lösungsmittel verwendet wird in einer Menge von 0,5 bis 51 Lösungsmittel pro 100 g des trockenen Pulvers von Asparagus Officinalis L.-Wurzeln und/oder - Sprossen, am allermeisten bevorzugt das Lösungmittel in einer Menge von 0,5 bis 51 Methanol und/oder Ethanol und/oder Aceton pro 100 g des trockenen Pulvers von Asparagus Officinalis-L.-Wurzeln und/oder -Sprossen verwendet wird.

24. Verfahren nach einem der Ansprüche 18 bis 23, worin in Schritt (c) das Rühren der Suspension ein Schütteln der Suspension ist, vorzugsweise ein Schütteln der Suspension für eine Zeit im Bereich von 10 bis 48 h und/oder Schütteln der Suspension bei einer Temperatur im Bereich von 10 bis 25 °C ist.

25. Verfahren nach einem der Ansprüche 18 bis 24, worin in Schritt (d) unlösliche und lösliche Bestandteile der Suspension getrennt werden durch wenigstens einen Schritt, der ausgewählt ist aus der Gruppe, die besteht aus Zentrifugieren, Dekantieren, Filtrieren und Kombinationen dieser Schritte.

26. Verfahren nach einem der Ansprüche 18 bis 25, worin in Schritt (e) das Lösungsmittel von der Lösung entfernt wird durch wenigstens einen Schritt, der ausgewählt ist aus der Gruppe, die besteht aus teilweisem Verdampfen des Lösungsmittels und Kristallisieren der gelösten Substanz, vollständigem Verdampfen des Lösungsmittels und Gewinnen der trockenen gelösten Substanz, Gefriertrocknen der Lösung und Kombinationen dieser Schritte.

27. Verfahren nach einem der Ansprüche 18 bis 26, worin in Schritt (i) ein Lösungsmittel verwendet wird, das eine Mischung aus Wasser und einem hohen Gehalt an wenigstens einer organischen Flüssigkeit, die mit Wasser mischbar ist, umfasst, vorzugsweise worin ein Lösungsmittel verwendet wird, das Wasser und einen hohen Gehalt an wenigstens einer organischen Flüssigkeit umfasst, die ausgewählt ist aus der Gruppe, die besteht aus geradkettigen oder verzweigten oder cyclischen Alkoholen mit 1 bis 6 Kohlenstoffatomen und geradkettigen oder verzweigten oder cyclischen Ketonen mit 3 bis 6 Kohlenstoffatomen, noch mehr bevorzugt worin ein Lösungsmittel verwendet wird, das Wasser und einen hohen Gehalt an einer organischen Flüssigkeit umfasst, die ausgewählt ist aus der Gruppe, die besteht aus geradkettigen oder verzweigten aliphatischen Alkoholen mit 1 bis 3 Kohlenstoffatomen und geradkettigen oder verzweigten aliphatischen Ketonen mit 3 bis 5 Kohlenstoffatomen, noch mehr bevorzugt worin ein Lösungsmittel verwendet wird, das Wasser und einen hohen Gehalt an Methanol und/oder Ethanol und/oder Aceton umfasst, noch mehr bevorzugt worin ein Lösungsmittel verwendet wird, das Wasser und mehr als 50 Vol-% Methanol und/oder Ethanol und/oder Aceton umfasst, noch mehr bevorzugt worin ein Lösungsmittel verwendet wird, das Wasser und 60 bis 90 Vol-% Methanol und/oder Ethanol und/oder Aceton umfasst, am meisten bevorzugt worin ein Lösungsmittel verwendet wird, das Wasser und 75 bis 95 Vol-% Methanol und/oder Ethanol und/oder Aceton umfasst, oder worin als Lösungsmittel eine rein organische Flüssigkeit verwendet wird, vorzugsweise eine organische Flüssigkeit, die ausgewählt ist aus der Gruppe, die besteht aus geradkettigen oder verzweigen oder cyclischen Alkoholen mit 1 bis 6 Kohlenstoffatomen und geradkettigen oder verzweigten oder cyclischen Ketonen mit 3 bis 6 Kohlenstoffatomen, noch mehr bevorzugt eine organische Flüssigkeit, die ausgewählt ist aus der Gruppe, die besteht aus geradkettigen oder verzweigten aliphatischen Alkoholen mit 1 bis 3 Kohlenstoffatomen und geradkettigen oder verzweigten aliphatischen Ketonen mit 3 bis 5 Kohlenstoffatomen, noch mehr bevorzugt eine organische Flüssigkeit, die ausgewählt ist aus der Gruppe, die besteht aus Methanol und/oder Ethanol und/oder Aceton, am meisten bevorzugt Ethanol verwendet wird.

28. Verfahren nach einem der Ansprüche 18 bis 27, worin in Schritt (ii) das Rühren der Suspension oder Lösung ein Schütteln der Suspension oder Lösung ist, vorzugsweise ein Schütteln der Suspension oder Lösung für eine Zeit im Bereich von 10 bis 48 h und/oder Schütteln der Suspension oder Lösung bei einer Temperatur im Bereich von 10 bis 25 °C ist.

29. Verfahren nach einem der Ansprüche 18 bis 24, worin in Schritt (iii) unlösliche und lösliche Bestandteile der Suspension getrennt werden durch wenigstens einen Schritt, der ausgewählt ist aus der Gruppe, die besteht aus Zentrifugieren, Dekantieren, Filtrieren und Kombinationen dieser Schritte.

30. Verfahren nach einem der Ansprüche 18 bis 29, worin in Schritt (iv) das Lösungsmittel von der Lösung entfernt wird durch wenigstens einen Schritt, der ausgewählt ist aus der Gruppe, die besteht aus teilweisem Verdampfen des Lösungsmittels und Kristallisieren der gelösten Substanz, vollständigem Verdampfen des Lösungsmittels und Gewinnen der trockenen gelösten Substanz, Gefriertrocknen der Lösung und Kombinationen dieser Schritte.

31. Verfahren nach einem der Ansprüche 18 bis 30, worin Asparagus Officinalis L.-Wurzeln als Ausgangsmaterial verwendet werden.

32. Verfahren nach einem der Ansprüche 18 bis 30, worin ein wässriger Rohextrakt aus Asparagus Officinalis-L.-Wurzeln, der durch die Schritte (a) bis (e) erhältlich ist, verwendet wird.

33. Verfahren nach einem der Ansprüche 18 bis 30, worin ein hydro-alkoholischer, vorzugsweise ein hydro-ethanolischer, noch mehr bevorzugt ein hydro-(20 bis 40 Vol-%-ethanolischer) Rohextrakt aus Asparagus Officinalis-L.-Wurzeln, der durch die Schritte (a) bis (e) erhältlich ist, verwendet wird.

34. Verfahren nach einem der Ansprüche 18 bis 30, worin Asparagus Officinalis L.-Sprossen als Ausgangsmaterial verwendet werden.

35. Verfahren nach einem der Ansprüche 18 bis 30, worin ein wässriger Rohextrakt aus Asparagus Officinalis-L.-Sprossen, der durch die Schritte (a) bis (e) erhältlich ist, verwendet wird.

36. Verfahren nach einem der Ansprüche 18 bis 30, worin ein hydro-alkoholischer, vorzugsweise ein hydro-ethanolischer, noch mehr bevorzugt ein hydro-(20 bis 40 Vol-%-ethanolischer) Rohextrakt aus Asparagus Officinalis-L.-Sprossen, der durch die Schritte (a) bis (e) erhältlich ist, verwendet wird.

37. Pharmazeutische Zubereitung oder Nahrungsergänzungsmittel, insbesondere pflanzliche diuretische Zubereitung, umfassend einen oder mehrere Saponinfreie(n) Extrakt(e) von Asparagus Officinalis L.-Wurzeln und/oder -Sprossen nach einem der Ansprüche 1 bis 17, gegebenenfalls zusammen mit einem oder mehreren pharmazeutisch annehmenbaren Trägerstoff(en), Hilfsstoff(en) und/oder Vehikeln.

38. Pharmazeutische Zubereitung oder Nahrungsergänzungsmittel, insbesondere pflanzliche diuretische Zubereitung nach Anspruch 37, umfassend den Saponin-freien Ethanol-unlöslichen Bestandteil des wässrigen oder des hydro-alkoholischen Rohextrakts von Asparagus Officinalis L.-Wurzeln und/oder -Sprossen.

39. Saponin-freier Extrakt aus Wurzeln und/oder Sprossen von Asparagus Officinalis L. nach einem der Ansprüche 1 bis 17 zur medizinischen Verwendung.

40. Verwendung eines oder mehrerer Extrakte(s) aus Asparagus Officinalis L.-Wurzeln und/oder -Sprossen nach einem der Ansprüche 1 bis 17 zur Herstellung eines Medikaments für die Unterstützung der Diurese, für die Unterstützung der Entschlackung des Körpers, für die Unterstützung der Reduzierung des Körpergewichts, für die Vorbeugung und/oder die Behandlung von Herzinsuffizienz, für die Vorbeugung und/oder die Behandlung von Bluthochdruck und/oder die Vorbeugung und/oder Behandlung von Herz-Hypertrophie.

## Revendications

1. Extrait sans saponine provenant de racines et/ou de pousses d'Asparagus Officinalis L., pouvant être obtenu par au moins les étapes consistant à :
a) broyer des racines et/ou des pousses sèches d'Asparagus Officinalis L. pour obtenir une poudre sèche ;
b) ajouter à la poudre sèche ainsi obtenue un solvant choisi parmi le groupe constitué par l'eau, au moins un liquide organique miscible à l'eau et des mélanges d'eau et d'au moins un liquide organique miscible à l'eau pour obtenir une suspension ;
c) agiter la suspension ainsi obtenue pendant une durée > 10 h et à une température n'exposant pas les constituants de ladite suspension au risque d'une dégradation biologique ;
d) séparer les constituants de la suspension non solubles dans ledit solvant de ces constituants solubles dans ledit solvant pour obtenir une fraction soluble dissoute dans le solvant et une fraction insoluble ; et
e) éliminer le solvant de la fraction soluble pour obtenir un extrait brut ; et
f) broyer facultativement l'extrait brut obtenu pour préparer un extrait brut de racines et/ou de pousses d'Asparagus Officinalis L. sous la forme d'une poudre ;
(i) ajouter au moins un solvant choisi parmi le groupe constitué par des liquides organiques miscibles à l'eau et des mélanges d'eau et d'au moins un liquide organique miscible à l'eau, dans lequel le solvant a une hydrophilie inférieure à celle du solvant utilisé pour la préparation de l'extrait brut, à la solution de constituants solubles de l'extrait brut obtenu à l'étape d) pour obtenir une solution ou une suspension ;
(ii) agiter la solution ou la suspension ainsi obtenue pendant une durée > 30 min et à une température n'exposant pas les constituants de ladite suspension au risque d'une dégradation biologique ;
(iii) séparer les phases liquides l'une de l'autre conformément à un paramètre approprié de celles-ci, et facultativement, de tout constituant insoluble pour obtenir une phase liquide avec un solvant ayant une hydrophilie relativement élevée et une phase liquide avec un solvant ayant une hydrophilie relativement plus faible et, facultativement une phase solide insoluble dans les deux phases liquides ;
(iv) éliminer, de la phase liquide avec le solvant ayant une hydrophilie relativement élevée, le solvant pour obtenir un résidu solide ; et
(v) sécher et broyer facultativement le résidu solide pour obtenir un extrait fractionné sans saponine à partir de racines et/ou pousses d'Asparagus Officinalis L..

2. Extrait sans saponine provenant de racines et/ou de pousses d'Asparagus Officinalis L., pouvant être obtenu par au moins les étapes consistant à :
a) broyer des racines et/ou des pousses sèches d'Asparagus Officinalis L. pour obtenir une poudre sèche ;
b) ajouter à la poudre sèche ainsi obtenue un solvant choisi parmi le groupe constitué par l'eau, au moins un liquide organique miscible à l'eau et des mélanges d'eau et d'au moins un liquide organique miscible à l'eau pour obtenir une suspension ;
c) agiter la suspension ainsi obtenue pendant une durée > 10 h et à une température n'exposant pas les constituants de ladite suspension au risque d'une dégradation biologique ;
d) séparer les constituants de la suspension non solubles dans ledit solvant de ces constituants solubles dans ledit solvant pour obtenir une fraction soluble dissoute dans le solvant et une fraction insoluble ; et
e) éliminer le solvant de la fraction soluble pour obtenir un extrait brut ; et
f) broyer facultativement l'extrait brut obtenu pour préparer un extrait brut de racines et/ou de pousses d'Asparagus Officinalis L. sous la forme d'une poudre ;
(i) ajouter au moins un solvant choisi parmi le groupe constitué par des liquides organiques miscibles à l'eau et des mélanges d'eau et d'au moins un liquide organique miscible à l'eau, dans lequel le solvant a une hydrophilie inférieure à celle du solvant utilisé pour la préparation de l'extrait brut, à une solution de l'extrait brut obtenu à l'étape e) et/ou à l'étape f) dans un solvant choisi parmi le groupe constitué par l'eau et le solvant qui était utilisé pour obtenir l'extrait brut aux étapes a) à d), pour obtenir une solution ou une suspension ;
(ii) agiter la solution ou la suspension ainsi obtenue pendant une durée > 30 min et à une température n'exposant pas les constituants de ladite suspension au risque d'une dégradation biologique ;
(iii) séparer les phases liquides l'une de l'autre conformément à un paramètre approprié de celles-ci, et facultativement, de tout constituant insoluble pour obtenir une phase liquide avec un solvant ayant une hydrophilie relativement élevée et une phase liquide avec un solvant ayant une hydrophilie relativement plus faible et, facultativement une phase solide insoluble dans les deux phases liquides ;
(v) éliminer, de la phase liquide avec le solvant ayant une hydrophilie relativement élevée, le solvant pour obtenir un résidu solide ; et
(vi) sécher et broyer facultativement le résidu solide pour obtenir un extrait fractionné sans saponine à partir de racines et/ou de pousses d'Asparagus Officinalis L..

3. Extrait sans saponine provenant de racines et/ou de pousses d'Asparagus Officinalis L., pouvant être obtenu par au moins les étapes consistant à :
a) broyer des racines et/ou des pousses sèches d'Asparagus Officinalis L. pour obtenir une poudre sèche ;
b) ajouter à la poudre sèche ainsi obtenue un solvant choisi parmi le groupe constitué par l'eau, au moins un liquide organique miscible à l'eau et des mélanges d'eau et d'au moins un liquide organique miscible à l'eau pour obtenir une suspension ;
c) agiter la suspension ainsi obtenue pendant une durée > 10 h et à une température n'exposant pas les constituants de ladite suspension au risque d'une dégradation biologique ;
d) séparer les constituants de la suspension non solubles dans ledit solvant de ces constituants solubles dans ledit solvant pour obtenir une fraction soluble dissoute dans le solvant et une fraction insoluble ; et
e) éliminer le solvant de la fraction soluble pour obtenir un extrait brut ; et
f) broyer facultativement l'extrait brut obtenu pour préparer un extrait brut de racines et/ou de pousses d'Asparagus Officinalis L. sous la forme d'une poudre ;
(i) ajouter au moins un solvant choisi parmi le groupe constitué par des liquides organiques miscibles à l'eau et des mélanges d'eau et d'au moins un liquide organique miscible à l'eau, dans lequel le solvant a une hydrophilie inférieure au solvant utilisé pour la préparation de l'extrait brut, à l'extrait brut obtenu à l'étape e) et/ou à l'étape f) pour obtenir une suspension ;
(ii) agiter la solution ainsi obtenue pendant une durée > 30 min et à une température n'exposant pas les constituants de ladite suspension au risque d'une dégradation biologique ;
(iii) séparer la phase liquide de tout constituant insoluble pour obtenir un résidu solide insoluble dans la phase liquide ; et
(iv) sécher et broyer facultativement le résidu solide pour obtenir un extrait fractionné sans saponine à partir de racines et/ou de pousses d'Asparagus Officinalis L..

4. Extrait sans saponine selon l'une quelconque des revendications 1 à 3, dans lequel à l'étape b) de l'eau est utilisée en tant que solvant, de préférence de l'eau en tant que solvant en une quantité de 0,1 à 10 L d'eau pour 100 g d'une poudre sèche de racines et/ou de pousses d'Asparagus Officinalis L., de manière davantage préférée de l'eau en tant que solvant en une quantité de 0,5 à 5 L d'eau pour 100 g de poudre sèche de racines et/ou de pousses d'Asparagus Officinalis L..

5. Extrait sans saponine selon l'une quelconque des revendications 1 à 3, dans lequel à l'étape b) il est utilisé en tant que solvant au moins un liquide organique miscible à l'eau et choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone, choisi de préférence dans le groupe constitué par les alcools aliphatiques à chaîne droite ou ramifiée comportant 1 à 6 atomes de carbone, de manière davantage préférée comportant 1 à 3 atomes de carbone, et les cétones aliphatiques à chaîne droite ou ramifiée comportant 3 à 5 atomes de carbone, de manière même davantage préférée choisi parmi le méthanol et l'éthanol et l'acétone et les mélanges de ceux-ci, de manière préférée entre toutes en une quantité de 0,5 à 5 L du solvant pour 100 g de la poudre sèche de racines et/ou de pousses d'Asparagus Officinalis L., de manière préférée entre toutes en une quantité de 0,5 à 5 L de méthanol et/ou d'éthanol et/ou d'acétone pour 100 g de la poudre sèche de racines et/ou de pousses d'Asparagus Officinalis L..

6. Extrait sans saponine selon l'une quelconque des revendications 1 à 3, dans lequel à l'étape b) il est utilisé en tant que solvant un mélange d'eau et d'au moins un liquide organique miscible à l'eau et choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone, de préférence un mélange d'eau et d'un liquide organique choisi parmi le groupe constitué par les alcools aliphatiques à chaîne droite ou ramifiée comportant 1 à 6 atomes de carbone, de manière davantage préférée comportant 1 à 3 atomes de carbone, et les cétones aliphatiques à chaîne droite ou ramifiée comportant 3 à 5 atomes de carbone, de manière encore davantage préférée un mélange d'eau et d'un liquide organique choisi parmi le méthanol et l'éthanol et l'acétone et les mélanges de ceux-ci, de manière encore davantage préférée un mélange d'eau et jusqu'à 50 % en volume de méthanol et/ou d'éthanol et/ou d'acétone, de manière encore davantage préférée un mélange d'eau et de 20 à 40 % en volume de méthanol et/ou d'éthanol et/ou d'acétone, de manière encore davantage préférée un mélange d'eau et de 25 à 35 % en volume de méthanol et/ou d'éthanol et/ou d'acétone, de manière préférée entre toutes ledit solvant en une quantité de 0,5 à 5 L du solvant pour 100 g de la poudre sèche de racines et/ou de pousses d'Asparagus Officinalis L., de manière préférée entre toutes ledit solvant en une quantité de 0,5 à 5 L de méthanol et/ou d'éthanol et/ou d'acétone pour 100 g de la poudre sèche de racines et/ou de pousses d'Asparagus Officinalis L..

7. Extrait sans saponine selon l'une quelconque des revendications 1 à 6, dans lequel l'étape c) est réalisée en secouant la suspension pendant une durée dans la gamme de 10 à 48 h et/ou en secouant ladite suspension à une température dans la gamme de 10 à 25 °C.

8. Extrait sans saponine selon l'une quelconque des revendications 1 à 7, dans lequel l'étape d) de séparation de constituants insolubles et solubles de la suspension est réalisée par au moins une étape choisie parmi le groupe constitué par la centrifugation, la décantation, la filtration et les combinaisons de ces étapes.

9. Extrait sans saponine selon l'une quelconque des revendications 1 à 8, dans lequel l'étape e) d'élimination du solvant de la solution est réalisée par au moins une étape choisie parmi le groupe constitué par l'évaporation partielle du solvant et la cristallisation du soluté, l'évaporation complète du solvant et la récupération du soluté sec, la lyophilisation de la solution et les combinaisons de ces étapes.

10. Extrait sans saponine selon l'une quelconque des revendications 1 à 9, dans lequel à l'étape (i) il est utilisé un solvant comprenant un mélange d'eau et d'une teneur élevée en au moins un liquide organique miscible à l'eau, de préférence un solvant comprenant de l'eau et une teneur élevée en au moins un liquide organique choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone, de manière davantage préférée un solvant comprenant de l'eau et une teneur élevée en un liquide organique choisi parmi le groupe constitué par les alcools aliphatiques à chaîne droite ou ramifiée comportant 1 à 3 atomes de carbone et les cétones aliphatiques à chaîne droite ou ramifiée comportant 3 à 5 atomes de carbone, de manière encore davantage préférée un solvant comprenant de l'eau et une teneur élevée en méthanol et/ou en éthanol et/ou en acétone, de manière encore davantage préférée un solvant comprenant de l'eau et plus de 50 % en volume de méthanol et/ou d'éthanol et/ou d'acétone, de manière encore davantage préférée un solvant comprenant de l'eau et 60 à 90 % en volume de méthanol et/ou d'éthanol et/ou d'acétone, de manière préférée entre toutes un solvant comprenant de l'eau et 75 à 95 % en volume de méthanol et/ou d'éthanol et/ou d'acétone ou en utilisant, en tant que solvant, un liquide purement organique, de préférence un liquide organique choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone, de manière davantage préférée un liquide organique choisi parmi le groupe constitué par les alcools aliphatiques à chaîne droite ou ramifiée comportant 1 à 3 atomes de carbone et les cétones aliphatiques à chaîne droite ou ramifiée comportant 3 à 5 atomes de carbone, de manière encore davantage préférée un liquide organique choisi dans le groupe constitué par le méthanol et/ou l'éthanol et/ou l'acétone, de manière préférée entre toutes l'éthanol.

11. Extrait sans saponine selon l'une quelconque des revendications 1 à 10, dans lequel l'étape (ii) est réalisée en secouant la suspension ou la solution, de préférence en secouant la suspension ou la solution pendant une durée dans la gamme de 30 à 90 min et/ou en secouant la suspension ou la solution à une température dans la gamme de 10 à 25 °C.

12. Extrait sans saponine selon l'une quelconque des revendications 1 à 11, dans lequel l'étape (iii) de séparation des constituants insolubles et solubles de la suspension est réalisée par au moins une étape choisie dans le groupe constitué par la centrifugation, la décantation, la filtration et les combinaisons de ces étapes.

13. Extrait sans saponine selon l'une quelconque des revendications 1 à 12, dans lequel l'étape (iv) est réalisée en éliminant le solvant de la solution par au moins une étape choisie parmi le groupe constitué par l'évaporation partielle du solvant et la cristallisation du soluté, l'évaporation complète du solvant et la récupération du soluté sec, la lyophilisation de la solution et les combinaisons de ces étapes.

14. Extrait sans saponine selon l'une quelconque des revendications 1 à 13, pouvant être obtenu à partir d'un extrait brut aqueux de racines d'Asparagus Officinalis L..

15. Extrait sans saponine selon l'une quelconque des revendications 1 à 13, pouvant être obtenu à partir d'un extrait brut hydroalcoolique, de préférence à partir d'un extrait brut hydroéthanolique, de manière davantage préférée à partir d'un extrait brut hydroéthanolique (20 à 40 % en volume) de racines d'Asparagus Officinalis L..

16. Extrait sans saponine selon l'une quelconque des revendications 1 à 13, pouvant être obtenu à partir d'un extrait brut aqueux de pousses d'Asparagus Officinalis L..

17. Extrait sans saponine selon l'une quelconque des revendications 10 à 13, pouvant être obtenu à partir d'un extrait brut hydroalcoolique, de préférence à partir d'un extrait brut hydroéthanolique, de manière davantage préférée à partir d'un extrait brut hydroéthanolique (20 à 40 % en volume) de pousses d'Asparagus Officinalis L..

18. Procédé de préparation d'un extrait sans saponine à partir de racines et/ou de pousses d'Asparagus Officinalis, comprenant au moins les étapes consistant à :
a) broyer des racines et/ou des pousses sèches d'Asparagus Officinalis L. pour obtenir une poudre sèche ;
b) ajouter à la poudre sèche ainsi obtenue un solvant choisi dans le groupe constitué par l'eau, au moins un liquide organique miscible à l'eau et des mélanges d'eau et d'au moins un liquide organique miscible à l'eau pour obtenir une suspension ;
c) agiter la suspension ainsi obtenue pendant une durée > 10 h et à une température n'exposant pas les constituants de ladite suspension au risque d'une dégradation biologique ;
d) séparer les constituants de la suspension non solubles dans ledit solvant de ces constituants solubles dans ledit solvant pour obtenir une fraction soluble dissoute dans le solvant et une fraction insoluble ; et
e) éliminer le solvant de la fraction soluble pour obtenir un extrait brut ; et
f) broyer facultativement l'extrait brut obtenu pour préparer un extrait brut de racines et/ou de pousses d'Asparagus Officinalis L. sous la forme d'une poudre ;
(i) ajouter au moins un solvant choisi parmi le groupe constitué par des liquides organiques miscibles à l'eau et des mélanges d'eau et d'au moins un liquide organique miscible à l'eau, dans lequel le solvant a une hydrophilie inférieure à celle du solvant utilisé pour la préparation de l'extrait brut, à la solution de constituants solubles de l'extrait brut obtenu à l'étape d) pour obtenir une solution ou une suspension ;
(ii) agiter la solution ou la suspension ainsi obtenue pendant une durée > 30 min et à une température n'exposant pas les constituants de ladite suspension au risque d'une dégradation biologique ;
(iii) séparer les phases liquides l'une de l'autre conformément à un paramètre approprié de celles-ci et, facultativement, de tout constituant insoluble pour obtenir une phase liquide avec un solvant ayant une hydrophilie relativement élevée et une phase liquide avec un solvant ayant une hydrophilie relativement plus faible et, facultativement une phase solide insoluble dans les deux phases liquides ;
(iv) éliminer, de la phase liquide avec le solvant ayant une hydrophilie relativement élevée, le solvant pour obtenir un résidu solide ; et
(v) sécher et broyer facultativement le résidu solide pour obtenir un extrait fractionné sans saponine à partir de racines et/ou pousses d'Asparagus Officinalis L..

19. Procédé de préparation d'un extrait sans saponine à partir de racines et/ou de pousses d'Asparagus Officinalis, comprenant au moins les étapes consistant à:
a) broyer des racines et/ou des pousses sèches d'Asparagus Officinalis L. pour obtenir une poudre sèche ;
b) ajouter à la poudre sèche ainsi obtenue un solvant choisi parmi le groupe constitué par l'eau, au moins un liquide organique miscible à l'eau et des mélanges d'eau et d'au moins un liquide organique miscible à l'eau pour obtenir une suspension;
c) agiter la suspension ainsi obtenue pendant une durée > 10 h et à une température n'exposant pas les constituants de ladite suspension au risque d'une dégradation biologique ;
d) séparer les constituants de la suspension non solubles dans ledit solvant de ces constituants solubles dans ledit solvant pour obtenir une fraction soluble dissoute dans le solvant et une fraction insoluble ; et
e) éliminer le solvant de la fraction soluble pour obtenir un extrait brut ; et
f) broyer facultativement l'extrait brut obtenu pour préparer un extrait brut de racines et/ou de pousses d'Asparagus Officinalis L. sous la forme d'une poudre ;
(i) ajouter au moins un solvant choisi parmi le groupe constitué par des liquides organiques miscibles à l'eau et des mélanges d'eau et d'au moins un liquide organique miscible à l'eau, dans lequel le solvant a une hydrophilie inférieure à celle du solvant utilisé pour la préparation de l'extrait brut, à une solution de l'extrait brut obtenu à l'étape e) et/ou à l'étape f) dans un solvant choisi parmi le groupe constitué par l'eau et le solvant qui était utilisé pour obtenir l'extrait brut dans les étapes a) à d), pour obtenir une solution ou une suspension ;
(ii) agiter la solution ou la suspension ainsi obtenue pendant une durée > 30 min et à une température n'exposant pas les constituants de ladite suspension au risque d'une dégradation biologique ;
(iii) séparer les phases liquides l'une de l'autre conformément à un paramètre approprié de celles-ci et, facultativement, de tout constituant insoluble pour obtenir une phase liquide avec un solvant ayant une hydrophilie relativement élevée et une phase liquide avec un solvant ayant une hydrophilie relativement plus faible et, facultativement une phase solide insoluble dans les deux phases liquides ;
(iv) éliminer, de la phase liquide avec le solvant ayant une hydrophilie relativement élevée, le solvant pour obtenir un résidu solide ; et
(v) sécher et broyer facultativement le résidu solide pour obtenir un extrait fractionné sans saponine à partir de racines et/ou pousses d'Asparagus Officinalis L..

20. Procédé de préparation d'un extrait sans saponine à partir de racines et/ou de pousses d'Asparagus Officinalis, comprenant au moins les étapes consistant à :
a) broyer des racines et/ou des pousses sèches d'Asparagus Officinalis L. pour obtenir une poudre sèche ;
b) ajouter à la poudre sèche ainsi obtenue un solvant choisi parmi le groupe constitué par l'eau, au moins un liquide organique miscible à l'eau et des mélanges d'eau et d'au moins un liquide organique miscible à l'eau pour obtenir une suspension ;
c) agiter la suspension ainsi obtenue pendant une durée > 10 h et à une température n'exposant pas les constituants de ladite suspension au risque d'une dégradation biologique ;
d) séparer les constituants de la suspension non solubles dans ledit solvant de ces constituants solubles dans ledit solvant pour obtenir une fraction soluble dissoute dans le solvant et une fraction insoluble ; et
e) éliminer le solvant de la fraction soluble pour obtenir un extrait brut ; et
f) broyer facultativement l'extrait brut obtenu pour préparer un extrait brut de racines et/ou de pousses d'Asparagus Officinalis L. sous la forme d'une poudre ;
(i) ajouter au moins un solvant choisi dans le groupe constitué par des liquides organiques miscibles à l'eau et des mélanges d'eau et d'au moins un liquide organique miscible à l'eau, dans lequel le solvant a une hydrophilie inférieure à celle du solvant utilisé pour la préparation de l'extrait brut, à l'extrait brut obtenu à l'étape e) et/ou à l'étape f) pour obtenir une suspension ;
(ii) agiter la suspension ainsi obtenue pendant une durée > 30 min et à une température n'exposant pas les constituants de ladite suspension au risque d'une dégradation biologique ;
(iii) séparer la phase liquide de tout constituant insoluble pour obtenir un résidu solide insoluble dans la phase liquide ; et
(iv) sécher et broyer facultativement le résidu solide pour obtenir un extrait fractionné sans saponine à partir de racines et/ou pousses d'Asparagus Officinalis L.

21. Procédé selon l'une quelconque des revendications 18 à 20, dans lequel à l'étape b) de l'eau est utilisée en tant que solvant, de préférence dans lequel de l'eau est utilisée en tant que solvant en une quantité de 0,1 à 10 L d'eau pour 100 g de poudre sèche de racines et/ou de pousses d'Asparagus Officinalis L., de manière davantage préférée dans lequel de l'eau est utilisée en tant que solvant en une quantité de 0,5 à 5 L d'eau pour 100 g de poudre sèche de racines et/ou de pousses d'Asparagus Officinalis L..

22. Procédé selon l'une quelconque des revendications 18 à 20, dans lequel à l'étape b) il est utilisé en tant que solvant au moins un liquide organique miscible à l'eau et choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone est utilisé en tant que solvant, de préférence dans lequel au moins un liquide organique choisi parmi le groupe constitué par les alcools aliphatiques à chaîne droite ou ramifiée comportant 1 à 6 atomes de carbone, de manière davantage préférée comportant 1 à 3 atomes de carbone, et les cétones aliphatiques à chaîne droite ou ramifiée comportant 3 à 5 atomes de carbone est utilisé en tant que solvant, de manière encore davantage préférée dans lequel au moins un liquide organique choisi parmi le méthanol et l'éthanol et l'acétone et les mélanges de ceux-ci est utilisé en tant que solvant, de manière préférée entre toutes dans lequel au moins un desdits solvants est utilisé en une quantité de 0,5 à 5 L du solvant pour 100 g de poudre sèche de racines et/ou de pousses d'Asparagus Officinalis L., de manière préférée entre toutes en une quantité de 0,5 à 5 L de méthanol et/ou d'éthanol et/ou d'acétone pour 100 g de poudre sèche de racines et/ou de pousses d'Asparagus Officinalis L..

23. Procédé selon l'une quelconque des revendications 18 à 20, dans lequel à l'étape b) il est utilisé en tant que solvant un mélange d'eau et d'au moins un liquide organique miscible à l'eau et choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone est utilisé en tant que solvant, de préférence dans lequel un mélange d'eau et d'un liquide organique choisi dans le groupe constitué par les alcools aliphatiques à chaîne droite ou ramifiée comportant 1 à 6 atomes de carbone, de manière davantage préférée comportant 1 à 3 atomes de carbone, et les cétones aliphatiques à chaîne droite ou ramifiée comportant 3 à 5 atomes de carbone, est utilisé en tant que solvant, de manière encore davantage préférée dans lequel un mélange d'eau et d'un liquide organique choisi parmi le méthanol et l'éthanol et l'acétone et les mélanges de ceux-ci est utilisé en tant que solvant, de manière encore davantage préférée dans lequel un mélange d'eau et de jusqu'à 50 % en volume de méthanol et/ou d'éthanol et/ou d'acétone est utilisé en tant que solvant, de manière encore davantage préférée dans lequel un mélange d'eau et de 20 à 40 % en volume de méthanol et/ou d'éthanol et/ou d'acétone, de manière encore davantage préférée un mélange d'eau et de 25 à 35 % en volume de méthanol et/ou d'éthanol et/ou d'acétone, est utilisé en tant que solvant ; de manière préférée entre toutes dans lequel ledit solvant est utilisé en une quantité de 0,5 à 5 L du solvant pour 100 g de poudre sèche de racines et/ou de pousses d'Asparagus Officinalis L., de manière préférée entre toutes ledit solvant est utilisé en une quantité de 0,5 à 5 L de méthanol et/ou d'éthanol et/ou d'acétone pour 100 g de poudre sèche de racines et/ou de pousses d'Asparagus Officinalis L..

24. Procédé selon l'une quelconque des revendications 18 à 23, dans lequel à l'étape c) l'agitation de la suspension est un secouement de la suspension, de préférence un secouement de la suspension pendant une durée dans la gamme de 10 à 48 h et/ou un secouement de la suspension à une température dans la gamme de 10 à 25 °C.

25. Procédé selon l'une quelconque des revendications 18 à 24, dans lequel à l'étape d) les constituants insolubles et solubles de la suspension sont séparés par au moins une étape choisie parmi le groupe constitué par la centrifugation, la décantation, la filtration et les combinaisons de ces étapes.

26. Procédé selon l'une quelconque des revendications 18 à 25, dans lequel à l'étape e) le solvant est éliminé de la solution par au moins une étape choisie parmi le groupe consistant en l'évaporation partielle du solvant et la cristallisation du soluté, l'évaporation complète du solvant et la récupération du soluté sec, la lyophilisation de la solution et les combinaisons de ces étapes.

27. Procédé selon l'une quelconque des revendications 18 à 26, dans lequel à l'étape (i) un solvant est utilisé comprenant un mélange d'eau et d'une teneur élevée en au moins un liquide organique miscible à l'eau, de préférence dans lequel un solvant comprenant de l'eau et une teneur élevée en au moins un liquide organique choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone est utilisé, de manière davantage préférée dans lequel un solvant comprenant de l'eau et une teneur élevée en un liquide organique choisi parmi le groupe constitué par les alcools aliphatiques à chaîne droite ou ramifiée comportant 1 à 3 atomes de carbone et les cétones aliphatiques à chaîne droite ou ramifiée comportant 3 à 5 atomes de carbone est utilisé, de manière encore davantage préférée dans lequel un solvant comprenant de l'eau et une teneur élevée en méthanol et/ou en éthanol et/ou en acétone est utilisé, de manière encore davantage préférée dans lequel un solvant comprenant de l'eau et plus de 50 % en volume de méthanol et/ou d'éthanol et/ou d'acétone est utilisé, de manière encore davantage préférée dans lequel un solvant comprenant de l'eau et 60 à 90 % en volume de méthanol et/ou d'éthanol et/ou d'acétone, de manière préférée entre toutes un solvant comprenant de l'eau et 75 à 95 % en volume de méthanol et/ou d'éthanol et/ou d'acétone est utilisé, ou dans lequel, en tant que solvant, un liquide purement organique est utilisé, de préférence dans lequel un liquide organique choisi parmi le groupe constitué par les alcools à chaîne droite ou ramifiée, ou cycliques comportant 1 à 6 atomes de carbone et les cétones à chaîne droite ou ramifiée, ou cycliques comportant 3 à 6 atomes de carbone est utilisé, de manière davantage préférée dans lequel un liquide organique choisi parmi le groupe constitué par les alcools aliphatiques à chaîne droite ou ramifiée comportant 1 à 3 atomes de carbone et les cétones aliphatiques à chaîne droite ou ramifiée comportant 3 à 5 atomes de carbone est utilisé, de manière encore davantage préférée dans lequel un liquide organique choisi dans le groupe constitué par le méthanol et/ou l'éthanol et/ou l'acétone, de manière préférée entre toutes l'éthanol, est utilisé.

28. Procédé selon l'une quelconque des revendications 18 à 27, dans lequel à l'étape (ii) l'agitation de la suspension ou de la solution est un secouement de la suspension ou de la solution, de préférence en secouant la suspension ou la solution pendant une durée dans la gamme de 30 à 90 min et/ou en secouant la suspension ou la solution à une température dans la gamme de 10 à 25 °C.

29. Procédé selon l'une quelconque des revendications 18 à 28, dans lequel à l'étape (iii) les constituants insolubles et solubles de la suspension sont séparés par au moins une étape choisie parmi le groupe constitué par la centrifugation, la décantation, la filtration et les combinaisons de ces étapes.

30. Procédé selon l'une quelconque des revendications 18 à 29, dans lequel à l'étape (iv) le solvant est éliminé de la solution par au moins une étape choisie dans le groupe constitué par l'évaporation partielle du solvant et la cristallisation du soluté, l'évaporation complète du solvant et la récupération du soluté sec, la lyophilisation de la solution et les combinaisons de ces étapes.

31. Procédé selon l'une quelconque des revendications 18 à 30, dans lequel les racines d'Asparagus Officinalis L. sont utilisées en tant que matière première.

32. Procédé selon l'une quelconque des revendications 18 à 30, dans lequel un extrait brut aqueux pouvant être obtenu par les étapes a) à e) des racines d'Asparagus Officinalis L. est utilisé.

33. Procédé selon l'une quelconque des revendications 18 à 30, dans lequel un extrait brut hydroalcoolique, de préférence un extrait brut hydroéthanolique, de manière davantage préférée un extrait brut hydroéthanolique (20 à 40 % en volume) pouvant être obtenu par les étapes a) à e) à partir de racines d'Asparagus Officinalis L. est utilisé.

34. Procédé selon l'une quelconque des revendications 18 à 30, dans lequel des pousses d'Asparagus Officinalis L. sont utilisées en tant que matière première.

35. Procédé selon l'une quelconque des revendications 18 à 30, dans lequel un extrait brut aqueux pouvant être obtenu par les étapes a) à e) à partir de pousses d'Asparagus Officinalis L. est utilisé.

36. Procédé selon l'une quelconque des revendications 18 à 30, dans lequel un extrait brut hydroalcoolique, de préférence un extrait brut hydroéthanolique, de manière davantage préférée un extrait brut hydroéthanolique (20 à 40 % en volume) pouvant être obtenu par les étapes a) à e) à partir de pousses d'Asparagus Officinalis L. est utilisé.

37. Préparation pharmaceutique ou complément nutritionnel, en particulier préparation diurétique végétale, comprenant un ou plusieurs extraits sans saponine provenant de racines et/ou de pousses d'Asparagus Officinalis L. selon l'une quelconque des revendications 1 à 17, facultativement avec une ou plusieurs substances supports, substances auxiliaires et/ou véhicules pharmacologiquement acceptables.

38. Préparation pharmaceutique ou complément nutritionnel, en particulier préparation diurétique végétale, selon la revendication 37, comprenant le composant insoluble dans l'éthanol sans saponine de l'extrait brut aqueux ou de l'extrait brut hydroalcoolique de racines et/ou de pousses d'Asparagus Officinalis L..

39. Extrait sans saponine provenant de racines et/ou de pousses d'Asparagus Officinalis L. selon l'une quelconque des revendications 1 à 17 pour une utilisation médicale.

40. Utilisation de l'un ou plusieurs des extraits sans saponine provenant de racines et/ou de pousses d'Asparagus Officinalis L. selon l'une quelconque des revendications 1 à 17, pour la fabrication d'un médicament pour favoriser une diurèse, pour favoriser une purification du corps, pour favoriser une réduction du poids corporel, pour la prévention et/ou le traitement d'une insuffisance cardiaque, pour la prévention et/ou le traitement d'une hypertension et/ou pour la prévention et/ou le traitement d'une hypertrophie du coeur.
